# EUROPÄISCHE PATENTSCHRIFT

(11) **EP 4 150 295 B1**
(45) Veröffentlichungstag und Bekanntmachung des Hinweises auf die Patenterteilung: **19.02.2025**
(21) Anmeldenummer: 21729212.7
(22) Anmeldetag: 10.05.2021
(51) Int. Cl.: G01B 15/04, G01B 21/04, G01N 23/046, A61B 6/58, A61B 6/03

(54) **COMPUTERIMPLEMENTIERTES VERFAHREN ZUR ZUSTANDSÜBERWACHUNG EINER VORRICHTUNG ZUR UNTERSUCHUNG VON OBJEKTEN UND ENTSPRECHENDES COMPUTERPROGRAMMPRODUKT**
COMPUTER-IMPLEMENTED METHOD FOR MONITORING THE STATUS OF A DEVICE FOR INVESTIGATING OBJECTS AND CORRESPONDING COMPUTER PROGRAM PRODUCT
PROCÉDÉ MIS EN OEUVRE PAR ORDINATEUR PERMETTANT LA SURVEILLANCE DE L'ÉTAT D'UN DISPOSITIF D'EXAMEN D'OBJETS ET PRODUIT PROGRAMME INFORMATIQUE CORRESPONDANT

(30) Priorität: 11.05.2020 DE 102020112651
(43) Veröffentlichungstag der Anmeldung: 22.03.2023
(73) Patentinhaber: Volume Graphics GmbH, 69115 Heidelberg (DE)
(72) Erfinder: GONDROM-LINKE, Sven, 69115 Heidelberg (DE); FLESSNER, Matthias, 69115 Heidelberg (DE); GÜNTHER, Thomas, 69115 Heidelberg (DE); POLIWODA, Christoph, 69115 Heidelberg (DE); SCHÜLLER, Sören, 69115 Heidelberg (DE); REINHART, Christof, 69115 Heidelberg (DE); HANDL, Daniela, 69115 Heidelberg (DE)
(74) Vertreter: Patentanwälte Olbricht Buchhold Keulertz
(86) Internationale Anmeldenummer: PCT/EP2021/062285
(87) Internationale Veröffentlichungsnummer: WO 2021/228747

(56) Entgegenhaltungen:
- WO-A1-2015/083031
- WO-A1-2018/072834
- WO-A1-2019/230040
- US-A1- 2019 200 948

## Beschreibung

Die Erfindung betrifft ein computerimplementiertes Verfahren zur Zustandsüberwachung einer Vorrichtung zur Untersuchung von Objekten.

Hergestellte Objekte können mittels Vorrichtungen zum Untersuchen von Objekten, z. B. mittels Computertomographie oder optische Sensoren wie Photogrammetrie oder Streifenprojektionssystemen, gemessen werden, um die Funktionsfähigkeit dieser Objekte zu erfassen bzw. zu beurteilen. Dabei können adaptive Messungen durchgeführt werden, die es ermöglichen, bereits vor der vollständigen Vermessung des Objekts eine Aussage über die Funktionsfähigkeit des Objekts zu treffen. Sobald eine hinreichende sichere Aussage getroffen werden kann, kann die adaptive Messung abgebrochen werden, um Zeit und damit Kosten zu sparen. Eine konventionelle Messung hingegen führt eine vollständige Vermessung des Objekts durch. Die Qualität der Aussage über die vermessenen Objekte hängt dabei von dem Funktionszustand der Vorrichtung zum Untersuchen von Objekten ab.

Bekannt sind Verfahren, bei denen eine Funktionsüberwachung eines Computertomographiesystems durchgeführt werden kann, wenn wiederkehrende Messaufgaben durchgeführt werden sollen. Mit diesen vorbekannten Verfahren, ist eine Überwachung der Funktionsfähigkeit von Computertomografiesystemen nur mit großem Aufwand bereitzustellen.

In dem Dokument WO 2019/230040 A1 ist folgendes offenbart: Dieses Röntgendickenmessgerät (12) ist mit einem Vorhersagedatenserver (14) verbunden und mit einer Röntgensteuerungsstromversorgung, einem Röntgengenerator und einer Erfassungseinheit ausgestattet. Der Vorhersagedatenserver (14) ist mit einer Erfassungseinheit (62), einer Vorhersageeinheit (64) und einer Speichereinheit (48) ausgestattet. Die Erfassungseinheit (62) erfasst von der Röntgendickenmessvorrichtung (12) Messinformationen (56), die mindestens eines der folgenden Elemente enthalten: einen Ansteuerspannungswert; einen Ansteuerstromwert; einen Röhrenspannungswert; einen Röhrenstromwert; und ein Erfassungssignal. Die Vorhersageeinheit (64) erzeugt Statistiken für Messinformationen (56), die von der Erfassungseinheit (62) erfasst wurden, als Vorhersagedaten (68) zur Diagnose von Anomalien in der Röntgendickenmessvorrichtung (12). Die Speichereinheit (48) speichert Vorhersagedaten (68), die von der Vorhersageeinheit (64) erzeugt wurden.

Aus dem Dokument US 2019/0200948 A1 ist folgendes offenbart: Die vorliegende Offenbarung bezieht sich auf Systeme und Verfahren zur Überwachung einer medizinischen Vorrichtung. Die medizinische Vorrichtung kann eine Röhre umfassen, die so konfiguriert ist, dass sie Strahlen erzeugt, und einen Detektor, der so konfiguriert ist, dass er von der Röhre emittierte Strahlen empfängt. Die Röhre kann ein Anodentarget und einen Glühfaden enthalten. Der Detektor kann eine Vielzahl von Erfassungseinheiten umfassen. Das Verfahren kann die Gewinnung von Abbildungsdaten umfassen, die durch den Detektor über die Erfassung der von der Röhre emittierten Strahlen erfasst werden. Das Verfahren kann auch die Bestimmung eines ersten Merkmalsparameters, der mit den Strahlen assoziiert ist, basierend auf den Abbildungsdaten umfassen. Das Verfahren kann ferner die Überwachung des medizinischen Geräts auf der Grundlage des ersten, den Strahlen zugeordneten Merkmalsparameters umfassen. Aufgabe der Erfindung ist es daher, ein computerimplementiertes Verfahren bereitzustellen, mit dem die Funktionsfähigkeit von Vorrichtungen zum Untersuchen von Objekten während adaptiver und konventioneller Messungen mit vergleichsweise geringem Aufwand überwacht wird. Hauptmerkmale der Erfindung sind in den Ansprüchen 1 und 14 angegeben. Ausgestaltungen sind Gegenstand der Ansprüche 2 bis 13.

In einem ersten Aspekt betrifft die Erfindung ein computerimplementiertes Verfahren zur Zustandsüberwachung einer Vorrichtung zur Untersuchung von Objekten, wobei die Untersuchung eines Objekts das Ermitteln von Messdaten mittels einer Messung des Objekts umfasst und während der Untersuchung des Objekts Betriebsdaten der Vorrichtung ermittelt werden, wobei das Verfahren die folgenden Schritte aufweist: Ermitteln von Messdaten des Objekts mittels der Vorrichtung; Ermitteln von Betriebsdaten der Vorrichtung während des Schritts Ermitteln von Messdaten des Objekts; Ermitteln mindestens eines Qualitätsparameters aus den Messdaten; Analysieren der Betriebsdaten und des mindestens einen Qualitätsparameters; und Ermitteln eines Zustandskennwerts basierend auf der Analyse der Betriebsdaten und des mindestens einen Qualitätsparameters zur Zustandsüberwachung der Vorrichtung, wobei der Zustandskennwert einen Zustand der Vorrichtung angibt.

Mit der Erfindung wird damit ein computerimplementiertes Verfahren bereitgestellt, bei dem aus einer Analyse von Betriebsdaten der Vorrichtung und eines Qualitätsparameters der Messdaten der Zustand der Vorrichtung mittels eines Zustandskennwerts während oder nach der Durchführung einer Messung des Objekts überwacht wird. D.h., während die Messung des Objekts noch läuft, werden bereits Betriebsdaten der Vorrichtung ermittelt und zusammen mit den bisher gesammelten Messdaten analysiert. Das Ergebnis dieser Analyse ist ein Zustandskennwert, der eine Aussage über den Zustand der Vorrichtung trifft. Die Messung des Objekts kann dabei eine adaptive Messung sein, die frühzeitig gestoppt werden kann, sobald genügend Daten über den Zustand des Objekts bereitstehen, und/oder anhand der vorliegenden Messdaten optimierte Aufnahmeparameter für die weitere Durchführung der Messung ermittelt. Weiter wird mit dem erfindungsgemäßen computerimplementierten Verfahren eine automatische Überwachung der Vorrichtung zur Untersuchung von Objekten bereitgestellt. Mit dem computerimplementierten Verfahren gemäß der Erfindung wird automatisch ein Fehlerzustand ermittelt, wenn die Vorrichtung zur Untersuchung von Objekten nicht mehr korrekt funktioniert, um während eines adaptiven oder konventionellen Messvorgangs Objekte mit genügend hoher Genauigkeit zu vermessen. Damit wird die von einem Benutzer benötigte Mitwirkung bei der Überwachung der Vorrichtung zur Untersuchung von Objekten verringert.

Für die Überwachung können sämtliche Informationen, die während des Betriebs der Vorrichtung zur Untersuchung von Objekten, zum Beispiel einem Computertomografiesystem, vorhanden und verfügbar sind, ausgewertet werden, selbst wenn sie auf den ersten Blick nicht hilfreich erscheinen. Durch die gleichzeitige Erfassung der Betriebsdaten der Vorrichtung zur Untersuchung von Objekten und der Messdaten über das Objekt mittels der Vorrichtung zur Untersuchung von Objekten können Informationen aus der Überwachung bei der adaptiven oder konventionellen Messung verwendet werden. Weiter wird durch die gleichzeitige Erfassung der Betriebsdaten und der Messdaten der adaptiven oder konventionellen Messung optimal überwacht und es können bei Bedarf Fehlerzustände und deren Ursachen identifiziert werden. Mit der Ermittlung des Zustandskennwerts wird dabei die Überwachung des Funktionszustandes der Vorrichtung während des adaptiven oder konventionellen Messvorgangs durchgeführt.

Der Zustandskennwert kann dabei zum Beispiel anzeigen, dass der Zustand der Vorrichtung in Ordnung oder gestört ist, d. h. ob ein Normalzustand der Vorrichtung oder ein Fehlerzustand vorliegt. Auch Zwischenzustände sind möglich, z. B. wenn einzelne Detektorpixel defekt sind, sog. Bad Pixel, was sich allerdings noch nicht signifikant auf die Messergebnisse auswirkt. In diesem Fall würde der Zustandskennwert anzeigen, dass eine gewisse, aber noch nicht problematische Abweichung von einem Normalzustand besteht, mit der die Vorrichtung noch nicht in den Fehlerzustand übergeht. In diesem Fall können frühzeitig Gegenmaßnahmen eingeleitet werden, um das Eintreten eines Fehlerzustandes zu verhindern.

Bei den Messdaten kann es sich um finale Messergebnisse, bei einer dimensionellen Messung, z. B. dem Durchmesser einer Bohrung, handeln. Weiter können Messdaten auch Rohdaten sein, die in der Messkette verarbeitet werden, im Falle einer durchstrahlenden Messung z. B. Durchstrahlungsbilder bzw. Projektionsdaten, rekonstruierte Volumendaten und/oder Oberflächendaten, im Falle einer Messung mittels Photogrammetrie oder Streifenprojektion z. B. Bilder der Messkamera oder errechnete Oberflächendaten.

Gemäß einem Beispiel kann der Schritt Ermitteln eines Zustandskennwerts den folgenden Unterschritt aufweisen: Vergleichen der Betriebsdaten und des mindestens einen Qualitätsparameters mit vordefinierten Vergleichswerten für die Betriebsdaten und den mindestens einen Qualitätsparameter zur Ermittlung eines Zustandskennwerts.

Dabei werden vordefinierte Vergleichswerte für die Betriebsdaten und den mindestens einen Qualitätsparameter verwendet, damit das computerimplementierte Verfahren entscheiden kann, ob ein Zustand der Vorrichtung bereits ein Fehlerzustand ist oder noch ein zu Normalzustand ist. Der Vergleich ist nicht darauf beschränkt, dass für einzelne Größen feststehende Vergleichswerte vordefiniert werden. Es kann auch so umgesetzt sein, dass mehrere Größen gemeinsam betrachtet werden. Zum Beispiel, wenn eine erste Größe A oder zweite Größe B einzeln um 5 % bis 10 % von einem jeweiligen Normalwert abweichen, ist dies noch nicht zwingend ein Fehlerzustand. Falls in diesem Beispiel jedoch beide Größen gleichzeitig um 5 % bis 10 % vom Nominalwert abweichen, kann dies als ein Fehlerzustand identifiziert werden. Die erste Größe A und die zweite Größe B können Betriebsdaten und/oder Qualitätsparameter sein.

Alternativ oder zusätzlich zu der Verwendung von vordefinierten Vergleichswerten kann ein Vergleich auf Basis von vorermittelten Musterdaten für die Betriebsdaten und die mindestens ein Qualitätsparameter durchgeführt werden, wobei die Musterdaten aus Trainingsdaten abgeleitet werden. Dabei kann der Schritt Ermitteln eines Zustandskennwerts zum Beispiel weiter den folgenden Unterschritt aufweisen: Vergleichen der Betriebsdaten und des mindestens einen Qualitätsparameters mit vorermittelten aus Trainingsdaten abgeleiteten Musterdaten für die Betriebsdaten und den mindestens einen Qualitätsparameter zur Ermittlung eines Zustandskennwerts.

Die vorermittelten Musterdaten dienen damit als eine Art Referenz für die während der Messung ermittelten Betriebsdaten und des mindestens einen Qualitätsparameters. Diese Trainingsdaten bzw. Musterdaten, können vorzugsweise dafür genutzt werden, um ein Verfahren des Machine Learnings, z. B. ein neuronales Netz, zu trainieren. Der Unterschritt wird dann von diesem Verfahren des Machine Learnings durchgeführt. Zur Erzeugung von Trainingsdaten können beispielsweise Messungen eines Referenzobjektes verwendet werden.

Die Trainingsdaten können daher aus unterschiedlichen Quellen gesammelt werden. So können z. B. lokal auf dem überwachten Computertomografiesystem Fehlerzustände identifiziert werden, z. B. durch ungewöhnlich hohe Messabweichungen bei der Messung eines Meisterteils. Dabei können dieser Fehlerzustand und die Charakteristika der überwachten Größen zu diesem Zeitpunkt in die Trainingsdaten aufgenommen werden. Weiter können auf die gleiche Weise Trainingsdaten von Computertomografiesystemen der gleichen oder zumindest ähnlichen Bauart zusammengetragen werden. Auf diese Weise wird die Datengrundlage vergrößert, während die Aussagekraft aufgrund der möglichen Unterschiede der Eigenschaften der Computertomografiesysteme teilweise eingeschränkt sein könnte. Weiter, mit den gleichen Vorteilen, können auch Daten von Computertomografiesystemen mit deutlich unterschiedlicher Konfiguration in die Trainingsdaten aufgenommen werden.

Um für die überwachten Daten aus unterschiedlichen Quellen eine gewisse Vergleichbarkeit zu erzeugen, können diese auf jeweils typische Wertebereiche normalisiert werden.

Eine gewisse Dauer vor oder auch nach dem identifizierten Fehlerzustand können auch der zeitliche Verlauf und die Werte der überwachten Größen in die Trainingsdaten aufgenommen werden.

Weiterhin können nicht nur Fehlerzustände in die Trainingsdaten aufgenommen werden, sondern auch funktionsfähige Zustände, um diese ebenfalls identifizieren bzw. von den Fehlerzuständen abgrenzen zu können.

Verschiedene Methoden zur Mustererkennung bzw. aus dem maschinellen Lernen können verwendet werden, um aus den überwachten Größen und den verfügbaren Trainingsdaten ableiten zu können, ob derzeit ein Fehlerzustand, und ggf. die Ursache, vorliegt bzw. bevorsteht. Diese können umfassen: Methoden der künstlichen Intelligenz, z. B. künstliche neuronale Netze, Deep Learning, Support Vector Machines, Bayes-Klassifikator, Nächste-Nachbarn-Klassifikation, usw. Die Wahl der verwendeten mindestens einen Methode erfolgt so, dass mittels der Trainingsdaten in der vergleichsweise großen Menge der teilweise unübersichtlichen, überwachten Daten, Muster von Fehlerzuständen identifiziert werden können.

Die Trainingsdaten können weiter beispielsweise aus Messdaten erzeugt werden, die mittels der Vorrichtung mittels mindestens einer Messung innerhalb eines vordefinierten Zeitintervalls vor und/oder nach dem Ermitteln von Messdaten eines Referenzobjekts ermittelt werden, wobei das Referenzobjekt eine bekannte Geometrie und/oder eine bekannte Eigenschaft aufweist.

Die Messungen, von denen die Trainingsdaten abgeleitet werden, müssen weder Messungen eines Referenzobjekts sein, noch die gleiche oder ähnliche Geometrie aufweisen wie das zu untersuchende bzw. untersuchte Objekt. Gemäß einem Beispiel kann Referenzobjekt die gleiche oder eine ähnliche Geometrie aufweisen wie das zu untersuchende bzw. untersuchte Objekt. Es müssen lediglich die Geometrie bzw. die Ergebnisse der Messungen dieses Referenzobjekts bekannt sein. Aus dem Vergleich der Messdaten und den Ergebnissen der Messung des Referenzobjekts kann abgeleitet werden, in welchem Zustand sich die Vorrichtung befindet. Größere Abweichungen sind in diesem Fall ein Indiz für einen Fehlerzustand. Neben der Geometrie können auch andere Eigenschaften des gemessenen Objekts relevant sein, z. B. Defekte im inneren des Referenzobjekts, Eigenschaften von ggf. verwendeten Faserverbundwerkstoffen wie Faserdurchmesser, Faserdichte und Faserorientierung, oder Materialeigenschaften wie die ggf. lokale Dichte.

Ein Referenzobjekt kann dabei auch als Meisterteil bezeichnet werden. Meisterteilmessungen werden bei einer Vielzahl von Computertomografiesystemen regelmäßig durchgeführt, um den Funktionszustand des Computertomografiesystems zu testen. Typische Wiederholraten solcher Messungen reichen von mehrfach täglich bis zu einmal im Monat.

Ein Meisterteil ist dabei ein Objekt mit bekannten geometrischen Eigenschaften. Dies können äußere wie auch innere geometrische Maße sowie Materialeigenschaften wie Dichte, Faserdichte, -dicke, -orientierung, Porositäten oder andere Eigenschaften sein. Um den Funktionszustand des Computertomografiesystems zu überprüfen, wird eine Messung dieses Teils durchgeführt und die, ggf. aufgabenspezifischen, Messergebisse werden mit den Referenzwerten oder Kalibrierwerten, die im Idealfall von einer Messung mit geringerer Messunsicherheit, z. B. mit einem genaueren Sensor, stammen, verglichen. Das Ausmaß der auftretenden Abweichungen ermöglicht Rückschlüsse darauf, ob das Computertomografiesystem sich in einem für die definierte Aufgabe funktionsfähigen Zustand befindet. Wenn das Computertomografiesystem diese Messaufgabe erfolgreich durchführen kann, kann davon ausgegangen werden, dass es in einem "guten" Zustand ist und auch andere ähnliche und teilweise auch weniger ähnliche Messaufgaben erfüllen kann. Um im Vergleich der Messergebnisse mit den Referenzmesswerten eine Aussage über den Zustand treffen zu können, werden meist Grenzwertintervalle für die Messabweichung definiert.

Diese Messungen können gemäß diesem Beispiel für die Trainingsdaten verwendet werden.

Die in die Trainingsdaten beizufügende bzw. zu korrelierende Information, ob ein Fehlerzustand vorlag, kann anhand der Messabweichungen der Meisterteilmessung abgeleitet werden. Hierbei kann beispielsweise die Abschätzung "guter" oder "schlechter" Zustand daran getroffen werden, ob definierte Grenzwertintervalle der Messabweichung nicht eingehalten werden. Weiterhin ist es aber auch möglich anstelle einer solchen "binären" Einordnung auch feiner aufgelöst den Zustand des Messsystems einzuschätzen, beispielsweise "im Mittel 47% der Messwerte innerhalb der erlaubten Grenzwertintervalle" oder "30% der geprüften Messgrößen außerhalb der Grenzwertintervalle".

Die jeweilige Information, ob ein Fehlerzustand vorliegt, wird den entsprechenden Messdaten der überwachten Größen zugeordnet. Hierfür können die Messdaten während der Messung des Meisterteils verwendet werden, da hieraus die analysierten Messabweichungen entstanden sind.

Wenn davon ausgegangen werden kann, dass sich der Zustand eines Computertomografiesystems in einer kürzeren Zeitspanne nicht signifikant ändert, können auch Messdaten von Messungen in einer gewissen zeitlichen Umgebung zur Meisterteilmessung in die Trainingsdaten mit der Zuordnung zu dem der Meisterteilmessung abgeleiteten Zustand aufgenommen werden. Dies hat den Vorteil, dass auch Daten von Arten von Messungen, die nicht durch das Meisterteil repräsentiert werden, in die Trainingsdaten einfließen, z. B. wenn das Meisterteil aus Aluminium ist, während die "zeitlich umliegenden" Messungen aus Kunststoff sind. Damit werden Trainingsdaten von Kunststoff-Messungen erzeugt. Wie groß diese "zeitliche Umgebung" sinnvollerweise gewählt werden kann, hängt von der Stabilität des Computertomografiesystems ab.

Insbesondere, wenn Daten in die Trainingsdaten einfließen, die vor der Meisterteilmessung erfasst wurden, in der später ggf. ein Fehlerzustand festgestellt wird, kann zum Beispiel anhand von Mustern in diesen Daten eine Vorhersage von in der Zukunft zu erwartenden Fehlerzuständen getroffen werden.

Weiter kann alternativ oder zusätzlich den einzelnen Meisterteilmessungen, insbesondere denen, in denen Fehlerzustände entdeckt wurden, auch noch die Ursache für die Abweichungen zugeordnet werden, damit trainiert werden kann, auch dies vorherzusagen. Dies kann auch manuell geschehen.

Der Schritt Ermitteln eines Zustandskennwerts kann weiter beispielsweise den folgenden Unterschritt aufweisen: Ermitteln, ob der Zustandskennwert einen Fehlerzustand der Vorrichtung anzeigt.

Mit diesem Unterschritt wird der Zustandskennwert ausgelesen, um zu erfassen, ob er einen Fehlerzustand der Vorrichtung anzeigt. Dies dient dazu, um den Fehlerzustand gegebenenfalls identifizieren zu können. Dabei kann die Identifikation des Fehlerzustands zum Beispiel anzeigen, welche der Betriebsdaten bzw. des mindestens einen Qualitätsparameters die Grundlage für den Fehlerzustand bilden. Auf diese Weise kann der Fehler bei der Vorrichtung zur Untersuchung von Objekten automatisch mit dem computerimplementierten Verfahren lokalisiert werden.

Hierbei bedeutet ein Fehlerzustand einen Zustand, in welchem die Genauigkeit der Messung so stark abnimmt, dass die durchzuführenden Messaufgaben nicht mehr sinnvoll durchführbar sind. Dies kann also messaufgabenspezifisch, z. B. anhand der Toleranzen, welche laut Messplan geprüft werden sollen, aber auch spezifisch für ein Computertomografiesystem, z. B. anhand einer globalen Spezifikation bzw. Mindestgenauigkeit, definiert sein. Je nach Definition kann dementsprechend eine moderate Verschlechterung des Zustands des Computertomografiesystems, welche nur eine leichte Verschlechterung der Messgenauigkeit nach sich zieht, bei einem Computertomografiesystem mit vergleichsweise schlechter Spezifikation bzw. bei Messaufgaben mit vergleichsweise großen Toleranzen, noch als kein Fehlerzustand bewertet werden. Trotzdem kann diese Verschlechterung identifiziert und eine erste Warnung ausgegeben oder Gegenmaßnahmen eingeleitet werden.

Gemäß einem weiteren Beispiel kann das Verfahren weiter den folgenden Schritt aufweisen, wenn der Zustandskennwert anzeigt, dass ein Fehlerzustand der Vorrichtung vorliegen könnte: Ermitteln von Messdaten eines Referenzobjekts mittels der Vorrichtung; und Analysieren der Messdaten des Referenzobjekts zum Ermitteln von Informationen, ob der Fehlerzustand der Vorrichtung vorliegt.

In diesem Beispiel wird ein Referenzobjekt mittels der Vorrichtung zur Untersuchung von Objekten vermessen, um Messdaten des Referenzobjekts zu ermitteln, wenn der Verdacht auf einen Fehlerzustand der Vorrichtung vorliegt. Eine Möglichkeit ist es, dass die laufende Messung unterbrochen wird, um ein Referenzobjekt zu vermessen. Alternativ kann auch bis zum Abschluss einer eventuell laufenden Messung gewartet werden, bis ein Referenzobjekt vermessen wird. Mit den ermittelten Messdaten des Referenzobjekts wird eine Analyse durchgeführt, um zu ermitteln, ob die Vorrichtung in einem Fehlerzustand ist.

D. h., wenn sich herausstellt, dass ein Fehlerzustand vorliegen könnte und anhand der derzeitigen Datenlage nicht ermittelt werden kann, ob auch tatsächlich ein Fehlerzustand vorliegt bzw. worin die Ursache des Fehlerzustandes liegt, kann eine Art Selbstdiagnose angefordert bzw. durchgeführt werden.

Hierfür wird ein geeignetes Referenzobjekt, als sog. Meisterteil bzw. Normal, im Idealfall mit bekannter Geometrie, bei geeigneten Aufnahmeparametern gemessen, um auf diese Weise Messdaten zu erzeugen, die zielgerichtet analysiert werden können, um mehr Informationen über die Ursachen des Fehlerzustands zu erhalten. Die Auswahl, welches Meisterteil bzw. Normal bei welchen Aufnahmeparametern gemessen werden sollte, um zielgerichtet möglichst viele neue Informationen bzw. die noch fehlenden Informationen zu generieren, kann im Rahmen des Verfahrens getroffen werden. So kann z. B., falls aufgrund der vorliegenden Daten noch mehrere Fehlerursachen möglich sind, eine Messung gewählt werden, welche zwischen genau diesen Ursachen differenzieren kann.

Wenn in einem Beispiel in den Volumendaten eine erhöhte Unschärfe festgestellt wird und es nicht klar beurteilt werden kann, ob dies an einem vergrößerten Brennfleck oder an einer fehlerhaften geometrischen Kalibrierung liegt, kann automatisch eine einzelne Durchstrahlung eines kleinen Objekts, z. B. einer kleinen Kugel oder einem Draht in großer geometrischer Vergrößerung durchgeführt werden. Eine Rekonstruktion ist dafür nicht nötig. Ist die Unschärfe dieser Projektion nicht erhöht, kann der Brennfleck als Ursache ausgeschlossen werden. In diesem Fall ist die fehlerhafte geometrische Kalibrierung die wahrscheinliche Ursache, da diese sich nicht auf die Unschärfe von Projektionen auswirkt.

In einem anderen Beispiel kann, wenn nicht klar ist, ob eine fehlerhafte geometrische Kalibrierung oder Drifteffekte die Ursache für einen Fehlerzustand sind, automatisch ein schneller, z. B. mit einer geringen Anzahl Projektionen, und ein langsamer, z. B. mit einer großen Anzahl Projektionen, Scan eines Objekts bei sonst gleichen Aufnahmeparametern durchgeführt werden. Tritt der Fehlerzustand nur beim langsamen Scan auf, ist die Ursache vermutlich ein Drifteffekt. Tritt das Problem beim schnellen Scan noch immer auf, ist die Ursache vermutlich kein Drifteffekt, sondern eine fehlerhafte geometrische Kalibrierung. Durch weitere Tests, z. B. bei verschiedenen Vergrößerungsstufen, kann das Verfahren dies ggf. weiter eingrenzen.

Diese Messungen können im Idealfall mit Hilfe eines Teilewechsels automatisch durchgeführt werden. Alternativ oder zusätzlich kann auch ein Benutzer angewiesen werden, diese Messung durchzuführen. Weiterhin stehen im Idealfall verschiedene Meisterteile bzw. Normale für diese Messung zur Verfügung.

Diese Schritte zur Selbstanalyse können bereits durchgeführt werden, wenn ein Verdacht auf einen bevorstehenden Fehlerzustand besteht.

In einem anderen Beispiel kann das Verfahren weiter den folgenden Schritt aufweisen: Ermitteln eines Fehlerursachenparameters zumindest aus dem Zustandskennwert, wobei der Fehlerursachenparameter eine mögliche Fehlerursache für einen Fehlerzustand angibt.

Mit dem Fehlerursachenparameter kann eine mögliche Ursache für einen Fehlerzustand ermittelt werden. Dabei kann für die Ermittlung des Fehlerursachenparameters weiter vorzugsweise der folgende Schritt durchgeführt werden: Vergleichen der Betriebsdaten und des mindestens einen Qualitätsparameters mit vordefinierten Vergleichswerten für die Betriebsdaten und den mindestens einen Qualitätsparameter zur Ermittlung, welche mögliche Fehlerursache vorliegt. Damit werden aus dem Vergleich der Betriebsdaten und des mindestens einen Qualitätsparameters mit vordefinierten entsprechenden Vergleichswerten diejenigen Betriebsdaten und Qualitätsparameter ermittelt, die für den Fehlerzustand verantwortlich sind. Auf diese Weise kann eine mögliche Fehlerursache für den Fehlerzustand ermittelt werden. Dies grenzt die Suche nach Ursachen des Fehlerzustandes für einen Benutzer ein und spart Zeit und Kosten.

Es wird bei einem Fehlerzustand daher nicht nur die durch den Zustandskennwert dargestellte Informationen ausgegeben, "dass etwas nicht stimmt", was alleine schon eine hilfreiche Information ist, da ein Benutzer nun zumindest weiß, dass eine Fehlersuche gestartet werden muss, sondern es zusätzlich der Hinweis gegeben, wo die Fehlersuche beginnen sollte. Im Idealfall kann bereits vor dem Auftreten eines Fehlerzustands vorhergesagt werden, dass in naher Zukunft ein Fehlerzustand auftreten wird, z. B., dass bei einer computertomographischen Vorrichtung die Röntgenquelle erste Schwankungen der Leistung zeigt, was durch eine größere Variation des Grauwertniveaus im Hintergrund der Projektionsdaten erkannt wurde. Es kann nach jenem Beispiel daher angenommen werden, dass in naher Zukunft größere Probleme der Röntgenquelle zu erwarten sind.

Einem Beispiel gemäß können aus den überwachten Größen geschlossen werden, ob ein Fehlerzustand vorliegt und ggf., was die Ursache hierfür sein könnte, indem zum Beispiel eine Tabelle oder Entscheidungsbaum verwendet wird.

Ein Beispiel kann dafür sein, für die zu überwachenden Größen bestimmte Grenzwerte vorzugeben. Dabei wird überprüft, ob diese Grenzwerte über- bzw. unterschritten werden. Dies ergibt einen ersten Hinweis auf einen möglichen Fehlerzustand. Anhand der Information, welche Grö-ßen die Grenzwerte nicht einhalten und in welcher Richtung und in welchem Ausmaß die Grenzwerte überschritten wird, können anhand von Vorwissen mögliche Fehlerursachen abgeleitet werden.

Zum Beispiel kann festgestellt werden, dass die Volumendaten ungewöhnlich unscharf sind, d. h. die Punktspreizfunktion ist ungewöhnlich breit. Gleichzeitig wurde allerdings festgestellt, dass in den Projektionsdaten keine unauffällig große Unschärfe festzustellen ist. Somit ist es unwahrscheinlich, dass ein vergrößerter Spot, z. B. aufgrund einer Defokussierung des Elektronenstrahls, die Ursache ist, da sich dies auch in den Projektionsdaten äußern würde. Eine mögliche Ursache wäre eine Fehljustierung des Systems, d. h. der Rekonstruktion der Volumendaten ist die Lage und Orientierung von Quelle, Detektor und Drehtisch im Raum nicht fehlerfrei bekannt. Daraus ergeben sich unscharfe Volumendaten. Dieses Ergebnis der Analyse kann an den Benutzer weitergegeben werden. Dies ist ein Beispiel für die Verwendung eines Entscheidungsbaums zur Ermittlung einer Fehlerursache.

Für die überwachten Größen könnten jeweils mögliche Fehlerursachen vorgegeben sein, welche zu einer Verletzung der Grenzwerte führen könnten. Auch bei einer Verletzung von Grenzwerten durch zwei oder mehreren Größen gleichzeitig könnten entsprechende mögliche Fehlerursachen vorgegeben sein. Auch andersrum ist es natürlich möglich, für die Fehlerursachen jeweils vorzugeben, für welche Größen typischerweise sie eine Verletzung der Grenzwerte verursachen würden. Dies sind Beispiele für die Verwendung von entsprechenden Tabellen zur Ermittlung einer Fehlerursache.

Diese Vorgaben könnten manuell vorgegeben sein. Es können jedoch auch für vergangene Fehlerzustände, ggf. mit bekannter Ursache, die entsprechenden Größen gespeichert werden, die außerhalb von Grenzwertintervallen waren, und auf diese Weise, ggf. automatisch, über die Zeit eine Datenbank aufgebaut werden.

In einem weiteren Beispiel müssen keine festen Grenzwerte definiert werden, sondern es kann mit Erfahrungswerten, die sich automatisch anpassen können, gearbeitet werden, um auf diese Weise Auffälligkeiten zu identifizieren. Auffälligkeiten können sein, wenn z. B. die Temperatur im Messvolumen 3°C über dem Mittelwert der letzten Woche liegt, oder wenn das Rauschen im Hintergrund der Projektionen zwei Standardabweichungen über dem Mittelwert des letzten Monats liegt.

Zusammengefasst ist diese Herangehensweise am ehesten für die Erkennung derjenigen Zusammenhänge zwischen überwachter Größe und Fehlerzustand (ggf. inkl. Ursache) geeignet, welche für einen Experten vergleichsweise offensichtlich und beschreibbar sind.

Dabei kann das Verfahren beispielhaft weiter den folgenden Schritt aufweisen, wenn der Fehlerursachenparameter nicht ermittelt werden konnte: Ermitteln von Messdaten eines Referenzobjekts mittels der Vorrichtung; und Analysieren der Messdaten des Referenzobjekts zum Ermitteln von Informationen zur Ermittlung des Fehlerursachenparameters.

Dieser Schritt kann alternativ oder zusätzlich durchgeführt werden. Wenn kein Fehlerursachenparameter ermittelt werden konnte wird damit ein Referenzobjekt mittels der Vorrichtung vermessen, um Referenzmessdaten zu ermitteln. Diese Referenzmessdaten werden analysiert um die Fehlerursache zu ermitteln. Die Fehlerursache kann dabei zum Beispiel aus den Abweichungen zwischen den Ergebnissen der Messdaten des Referenzobjekts und den Messdaten des zu messenden Objekts zu bekannten Referenzwerten ermittelt werden. Analog können auch die Betriebsdaten analysiert werden. Vorteilhaft an der Verwendung eines Referenzobjekts ist, dass hierfür typischerweise mehr bzw. genauere Referenzwerte verfügbar sind. Damit kann das computerimplementierte Verfahren auch bei einem in vorherigen Schritten nicht ermittelbaren Fehlerursachenparameter mit diesem Schritt einen Fehlerursachenparameter ermitteln und eine Fehlersuche für einen Benutzer erleichtern.

Weiter kann zum Beispiel, wenn im Schritt Ermitteln eines Fehlerursachenparameters ein Fehlerursachenparameter ermittelt wird, der anzeigt, dass eine geometrische Kalibrierung der Vorrichtung einen Genauigkeitswert aufweist, der außerhalb eines vordefinierten Genauigkeitswertintervalls angeordnet ist, das Verfahren weiter folgenden Schritt aufweisen: Kalibrieren der Vorrichtung.

Eine Kalibrierung der Vorrichtung wird gemäß diesem Beispiel durchgeführt, wenn der Fehlerursachenparameter anzeigt, dass die geometrische Kalibrierung der Vorrichtung fehlerhaft ist, d.h. ein Genauigkeitswert aufweist, der außerhalb eines vordefinierten Genauigkeitsintervalls angeordnet ist. D.h., wenn mit dem computerimplementierten Verfahren herausgefunden wird, dass die Fehlerursache die fehlerhafte Kalibrierung der Vorrichtung ist, bewirkt das computerimplementierte Verfahren automatisch eine Kalibrierung der Vorrichtung, um den Genauigkeitswert für die geometrische Kalibrierung der Vorrichtung zu erhöhen, sodass der Genauigkeit wieder innerhalb des vordefinierten Genauigkeitswertintervalls liegt. Das Genauigkeitswertintervall kann dabei zum Beispiel zwischen 95 % und 100 % liegen, wobei 100 % die maximale Genauigkeit der geometrischen Kalibrierung darstellt. Es kann jedoch jedes beliebige Intervall zwischen 0 % und 100 % gewählt werden.

Wenn der Fehlerzustand anzeigt, dass die geometrische Kalibrierung beeinträchtigt ist, wird eine erhöhte Messunsicherheit zu erwarten sein. Das Genauigkeitsintervall kann hinsichtlich allgemeiner Anforderungen oder hinsichtlich einer spezifischen Messaufgabe definiert werden. Für dimensionelle Messungen kann dabei die Unsicherheit der Messung selbst abgeschätzt werden, z. B. anhand des Fehlers der Voxelgröße. Für die Analyse von Poren können Grenzwerte für die Unschärfe der Daten definiert werden.

Sollte sich auf diese Weise herausstellen, dass der Funktionszustand des Computertomografiesystems nicht ausreichend ist, kann automatisch ein sog. Einmessvorgang zur geometrischen Kalibrierung angefordert bzw. durchgeführt werden. Dies kann bedeuten, dass ein Benutzer aufgefordert wird, einen solchen Einmessvorgang durchzuführen. Falls möglich, kann dieser durch das Verfahren durchgeführt werden. Ein Einmessvorgang bedeutet meist, dass ein spezielles Normal, das meist aus einer oder mehreren Kugeln bestehet, im Strahlengang, ggf. in verschiedenen Positionierungen, positioniert wird und durchstrahlt wird. Anhand des Durchstrahlungsbildes kann, z. B. anhand der abgebildeten Positionen der Kugeln, die Geometrie des Computertomografiesystems erfasst werden. Bei axialer CT lässt sich dies im Allgemeinen durch neun Freiheitsgrade modellieren: Position und Orientierung von Detektor und Position der Röntgenquelle jeweils bzgl. des Drehtisches. Bei freien Trajektorien kann diese geometrische Kalibrierung auch deutlich komplexer sein, mit einer größeren Anzahl von Freiheitgraden. Die Erfassung dieser Geometrie wird als geometrische Kalibrierung bezeichnet. Auf dieser Geometrie kann die Rekonstruktion so durchgeführt bzw. parametrisiert werden, dass möglichst fehlerfreie und scharfe Volumendaten berechnet werden können.

Dieser Einmessvorgang kann komplett automatisch durchgeführt werden, z. B. indem das verwendete Normal durch einen vom Messsystem steuerbaren Teilewechsler im Messvolumen bzw. auf dem Drehtisch positioniert wird.

Eine Kalibrierung kann zum Beispiel auch prophylaktisch durchgeführt werden, wenn ein Fehlerzustand der Vorrichtung angezeigt wird. D.h., auch wenn unbekannt ist was die Ursache des Fehlers ist, d.h. wenn der Fehlerursachenparameter nicht ermittelt werden konnte, kann vorsorglich eine Kalibrierung der Vorrichtung durchgeführt werden. Mit der vorsorglichen Kalibrierung der Vorrichtung kann zumindest ausgeschlossen werden, dass die geometrische Kalibrierung der Vorrichtung die Fehlerursache ist, wenn der nach der Kalibrierung neu ermittelte Zustandskennwert weiterhin einen Fehlerzustand anzeigt.

Es kann allerdings auch, komplett ohne reales Normal, eine geometrische Kalibrierung anhand der erfassten Projektionsdaten einer normalen Messung durchgeführt werden. Dabei kann z. B. anhand der vorliegenden Messdaten bzw. Projektionen die geometrische Kalibrierung ermittelt werden, die am besten zu den Daten passt. Dies kann auch für bereits durchgeführte Messungen nachträglich durchgeführt werden.

Weiter kann die Vorrichtung zum Beispiel eine automatische Objektwechseleinheit aufweisen, wobei das Verfahren weiter den folgenden Schritt aufweist: Einwechseln eines Referenzobjekts für das Objekt in die Vorrichtung zur Untersuchung von Objekten zum Ermitteln von Messdaten über das Referenzobjekt und/oder Einwechseln eines Kalibrierobjektes für das Objekt in die Vorrichtung zur Untersuchung von Objekten zum Kalibrieren der Vorrichtung zur Untersuchung von Objekten.

In einem Beispiel weist die Objektwechseleinheit einen Roboterarm, der ein Objekt, ggf. inkl. einer Halterung für das Objekt, greifen und im Strahlengang oder auf dem Drehtisch platzieren kann.

In einem anderen Beispiel weist die Objektwechseleinheit mindestens einen Drehtisch auf, der von unten an eine Batterie von Objekten bzw. Halterungen heranfahren kann und, ggf. mit einer geeigneten Halterung, die Objekte aufnehmen kann. In keinem der Beispiele ist eine Aktion eines Benutzers erforderlich.

Beispielgemäß kann das Verfahren weiter die folgenden Schritte aufweisen: Durchführen zumindest des Schritts Ermitteln von Messdaten des Objekts mit einem ersten Objekt, wobei die Messung eine durchstrahlende Messung ist; Durchführen zumindest des Schritts Ermitteln von Messdaten des Objekts mit einem zweiten Objekt, wobei die Messung eine durchstrahlende Messung ist; Ermitteln von Aufnahmeparametersätzen der Vorrichtung, die für die Ermittlung von Messdaten für das erste Objekt und für die Ermittlung von Messdaten für das zweite Objekt identisch sind; Ermitteln von ersten Projektionsdarstellungen aus den Messdaten für das erste Objekt mittels der ermittelten Aufnahmeparametersätze und Ermitteln von zweiten Projektionsdarstellungen aus den Messdaten für das zweite Objekt mittels der ermittelten Aufnahmeparametersätze; und Analysieren mindestens eines ersten Qualitätsparameters, der einer den ersten Projektionsdarstellungen zugeordnet ist, und mindestens eines zweiten Qualitätsparameters, der mindestens einer der zweiten Projektionsdarstellungen zugeordnet ist, auf Unterschiede. In einem alternativen oder zusätzlichen Beispiel kann das Verfahren weiter die folgenden Schritte aufweisen, wenn ein Aufnahmeparametersatz eines ersten Objekts und ein Aufnahmeparametersatz eines zweiten Objekts zumindest teilweise nicht identisch sind: Definieren von Aufnahmeparametersätzen der Vorrichtung für die Ermittlung von Messdaten; Durchführen zumindest des Schritts Ermitteln von Messdaten des Objekts mit dem ersten Objekt mit den definierten Aufnahmeparametersätzen zum Ermitteln von ersten Projektionsdarstellungen, wobei die Messung eine durchstrahlende Messung ist; Durchführen zumindest des Schritts Ermitteln von Messdaten des Objekts mit einem zweiten Objekt, mit den definierten Aufnahmeparametersätzen zum Ermitteln von zweiten Projektionsdarstellungen, wobei die Messung eine durchstrahlende Messung ist; und Analysieren mindestens eines ersten Qualitätsparameters, der einer der ersten Projektionsdarstellungen zugeordnet ist, und mindestens eines zweiten Qualitätsparameters, der mindestens einer der zweiten Projektionsdarstellungen zugeordnet ist, auf Unterschiede; wobei eine Geometrie des zweiten Objekts innerhalb eines vordefinierten Toleranzintervalls von einer Geometrie des ersten Objekts abweicht.

Durchstrahlende Messungen können z. B. Röntgenmessungen, z. B. Radiographie oder Computertomographie sein.

Aufnahmeparameter einer Projektion können die Durchstrahlungsgeometrie der Projektion sein und/oder Einstelloptionen, welche bei der Durchstrahlung eines Objektes eingestellt werden können, beispielsweise Strom und Spannung der Röntgenquelle, die Belichtungszeit oder der gewählte Filter.

Eine Durchstrahlungsgeometrie beschreibt die Richtung, in der das Objekt bei der durchstrahlenden Messung durchstrahlt wird, die Position des durchstrahlten Bereichs und die Vergrößerung. Bei einer Röntgenmessung bzw. Durchstrahlung wird diese Geometrie daher durch die Lage der Röntgenquelle und des Detektors relativ zu dem Messobjekt beschrieben, woraus sich neun geometrische Freiheitsgrade ergeben. Für die Translation ergeben sich jeweils drei Freiheitsgrade für die Röntgenquelle und den Detektor, für die Rotation ergeben sich drei weitere Freiheitsgrade für den Detektor. Eine Durchstrahlungsgeometrie kann bzgl. des Objektes oder bzgl. der Vorrichtung zum Untersuchen von Objekten definiert sein.

Bei dem Schritt Analysieren mindestens eines ersten Qualitätsparameters, der einer der ersten Projektionsdarstellungen zugeordnet ist, und mindestens eines zweiten Qualitätsparameters, der mindestens einer der zweiten Projektionsdarstellungen zugeordnet ist, auf Unterschiede, können nicht nur Qualitätsparameter der Projektionsdarstellungen selbst, sondern beispielsweise auch Qualitätsparameter von aus den Projektionsdarstellungen rekonstruieren Volumendaten analysiert werden.

Es werden damit Messungen überwacht, bei denen zumindest teilweise unterschiedliche Aufnahmeparameter, zum Beispiel teilweise unterschiedliche Durchstrahlungsgeometrien bzw. Trajektorien für Messobjekte gleicher bzw. ähnlicher Geometrie, gefahren werden. In einer ersten Alternative wird für die Analyse des Funktionszustandes zwischen jeweils mindestens zwei Messungen eine Menge von Projektionsdarstellungen identifiziert, für die die Durchstrahlungsgeometrie und die restlichen Aufnahmeparameter identisch waren. Diese werden verglichen und ausgewertet, um einen Unterschied der Qualitätsparameter der beiden Messungen zu ermitteln. In der zweiten Alternative des Beispiels werden gezielt Projektionsdarstellungen mit gleicher Durchstrahlungsgeometrie und gleichen Einstellungen aufgenommen und analysiert.

Damit können ein Bezug bzw. eine gemeinsame Datenbasis zwischen unterschiedlichen Messungen, insbesondere adaptiven Messungen, hergestellt werden.

In der zweiten Alternative wird durch das gezielte Erstellen von gleichen Projektionen die Vergleichbarkeit zwischen einer großen Anzahl von Messungen hergestellt. Dafür wird ein zusätzlicher Zeitaufwand in Kauf genommen. Konventionelle Verfahren zur Überwachung, die diese Daten analysieren und z. B. nach Drifts untersuchen, können hierauf verwendet werden. Es können hierbei entweder die jeweils gleichen Projektionen direkt verglichen werden, oder auf Grundlage einer Rekonstruktion aus jeweils gleichen Projektionen Volumendaten ermittelt und analysiert werden. Zur Analyse dieser Daten für die Überwachung können die bereits oben genannten Verfahren genannt werden.

In der ersten Alternative ist wird ein solcher Vergleich jeweils zwischen mindestens zwei Messungen ermöglicht, wobei diese Messungen zeitlich "benachbart" sein können. Ein Vergleich ist somit zunächst nur für eine vergleichsweise kleine Anzahl von Messungen möglich. Wenn auf diese Weise beispielsweise zwischen Messungen A und B sowie für B und C ein Vergleich eines Qualitätsparameters bekannt ist, kann zumindest qualitativ ein Vergleich von A und C durchgeführt werden, hierfür sollten ein Qualitätsparameter verwendet werden, der in dieser Hinsicht eine gewisse Transitivität aufweist. Auf diese Weise können viele verschiedene Messungen "verkettet" verglichen bzw. überwacht werden und somit eine langfristige Überwachung durchgeführt werden, auch wenn jede einzelne Messung nur mit vergleichsweise wenigen Messungen direkt verglichen werden kann. Auch diese Analyse kann auf Basis von Projektionen und/oder von rekonstruierten Volumendaten durchgeführt werden.

Beide Alternativen erfordern, dass die Messobjekte der einzelnen Messungen mindestens eine ähnliche Geometrie aufweisen, z. B. eine gleiche Soll-Geometrie.

Eine Kombination dieser beiden Alternativen wäre es, bei der Messung gewisse Standard-Projektionen zu definieren, aus welchen für jede Messung flexibel die benötigten ausgewählt werden können. Da auf diese Weise die Auswahl der "erlaubten" Projektionen eingeschränkt wird. wenn genügend viele Standard-Projektionen definiert sind, ohne die Flexibilität stark einzuschränken, steigt die Wahrscheinlichkeit, dass zwei Messungen verglichen werden können bzw. es steigt die mittlere Anzahl der Projektionen, welche zwischen zwei Messungen vergleichbar sind. Diese Standard-Projektionen können zudem bei einem Einmessvorgang gesondert bzw. gezielt eingemessen werden, um die Genauigkeit zu erhöhen. Dies ist insbesondere vorteilhaft bei Roboter-CT, bei der die Positioniergenauigkeit vergleichsweise gering, aber die "Auswahl" der Durchstrahlungsrichtungen extrem groß ist.

Das Verfahren kann in einem weiteren Beispiel die folgenden Schritte aufweisen: Durchführen zumindest des Schritts Ermitteln von Messdaten des Objekts mit einem ersten Objekt, wobei die Messung eine durchstrahlende Messung ist; Durchführen zumindest des Schritts Ermitteln von Messdaten des Objekts mit einem zweiten Objekt, wobei die Messung eine durchstrahlende Messung ist, wobei eine andere Durchstrahlungsgeometrie verwendet wird als für das erste Objekt; Ermitteln von Projektionsdarstellungen aus den Messdaten des ersten Objekts; Ermitteln von Projektionsdarstellungen aus den Messdaten des zweiten Objekts; Vergleichen mindestens einer Unschärfe zwischen den Projektionsdarstellungen des ersten Objekts und den Projektionsdarstellungen des zweiten Objekts; und Analysieren der mindestens einen Unschärfe zur Ermittlung des Zustandskennwerts.

In diesem Beispiel werden Messungen überwacht, bei denen unterschiedliche Durchstrahlungsgeometrien bzw. Trajektorien gefahren werden, wie es beispielsweise bei adaptiven Messungen der Fall sein kann. Aus den Projektionsdaten wird die Unschärfe der Abbildung ermittelt und diese zur Überwachung des Funktionszustandes analysiert.

Hierfür ist es vorteilhaft, wenn Projektionsdarstellungen gleicher Einstelloptionen verglichen werden, wobei sich die Durchstrahlungsgeometrie allerdings unterscheiden kann. Bezüglich der Durchstrahlungsgeometrie kann es vorteilhaft sein, wenn Projektionen gleicher oder ähnlicher geometrischer Vergrößerung verglichen werden, wobei sich die anderen geometrischen Parameter unterscheiden können. Damit wird z. B. bei Röntgenaufnahmen sichergestellt, dass sich die durch die Ausdehnung des Röntgenspots verursachte Unschärfe ähnlich in den Messdaten äußert, was einen belastbareren Vergleich ermöglicht.

Der Vorteil der Analyse der Unschärfe in den Projektionsbilddaten ist, dass diese über viele Messungen vergleichbar ist, unabhängig davon, wie die Geometrie des durchstrahlten Objektes ist bzw. aus welcher Richtung es durchstrahlt wird. Diese restlichen zwischen den Messungen variierenden Einflussgrößen, z. B. die Aufnahmeparameter, insbesondere die daraus resultierende Größe des Brennflecks, und die geometrische Vergrößerung, lassen sich vergleichsweise einfach quantifizieren und ihr Einfluss auf die Unschärfe der Projektionen zurückrechnen.

Es ist allerdings nicht trivial, die durch das Abbildungssystem ausgelöste Unschärfe bzw. die Punktspreizfunktion ausgelöste Unschärfe in den Projektionsdaten zu ermitteln. Problematisch ist, dass die Durchstrahlung keine scharfe Kante von hell zu dunkel in den Projektionen ausbildet, da der Grauwert abhängig von der Durchstrahlungslänge ist. Wird ein Objekt durchstrahlt, steigt die Durchstrahlungslänge meist kontinuierlich an. Wird z. B. eine Kugel durchstrahlt, weist das Grauwertprofil von den Rändern der Kugel zu deren Mittelpunkt einen Gradienten auf. Dieser Gradient suggeriert eine Unschärfe, die jedoch durch die Geometrie des durchstrahlten Objekts verursacht wird und nicht durch die Unschärfe des Computertomografiesystems selbst, die ermittelt werden soll. Ein realer Grauwertverlauf ergibt sich in erster Näherung aus den durch das Objekt verursachten, theoretischen Grauwertverlauf, welcher mit der Punktspreizfunktion des Abbildungssystems gefaltet wird. Diese Punktspreizfunktion ist allerdings im Vergleich zu den Grauwertverläufen, welche durch das Objekt verursacht werden, extrem klein. Dies macht es schwer, diese beiden Effekte zu trennen, insbesondere, wenn die Geometrie des durchstrahlen Objektes nicht genau bekannt ist. Wenn der vom Objekt verursachte theoretische Grauwertverlauf eine scharfe Kante bildet, kann allerdings die durch die Punktspreizfunktion ausgelöste Unschärfe leichter ermittelt werden. Dieser Fall tritt aber selten auf, da viele Flächen entweder nicht ausreichend eben sind und gewisse Abweichungen aufweisen, die wiederum eine falsche Unschärfe verursachen, und auch nicht exakt bekannt ist, in welchen, wenn überhaupt, Projektionen Ebenen entsprechend "streifend" durchstrahlt werden. Die Identifikation der auszuwertenden Bereiche ist daher nicht trivial.

Falls in einem Beispiel die Geometrie des Messobjektes prinzipiell bekannt ist, z. B. durch Vorwissen wie einer Darstellung des Objekts mit CAD, kann eine Durchstrahlungsrichtung gezielt angefahren werden, in welchen sich eine möglichst scharfe Kante in den Durchstrahlungsbildern ausbildet, welche hinsichtlich der Unschärfe analysiert werden kann. Hierbei kann auch Vorwissen über die durch die Geometrie verursachte zu erwartende Unschärfe genutzt werden und hieraus aus der gemessenen Unschärfe der Einfluss bzw. die Unschärfe des Messsystems separiert werden.

Falls in einem anderen Beispiel die Geometrie vorab nicht bekannt ist, kann eine Auswertung der, ggf. vorläufigen, Messdaten durchgeführt werden, z. B. eine Rekonstruktion und eine Oberflächenbestimmung, um die Geometrie zu bestimmen. Analog können auf diese Weise geeignete Bereiche in den Projektionsdaten identifiziert werden, welche geeignete Kantenübergänge aufweisen.

In einem weiteren Beispiel können die auftretenden Ortsfrequenzen, inkl. jeweiliger Amplitude bzw. Kontrast in allen Projektionen analysiert bzw. statistisch ausgewertet werden. In einem Histogramm könnte beispielsweise der Peak bei der höchsten Ortsfrequenz, der klar zu erkennen ist, ein Maß für die Unschärfe sein. Dies können z. B. die Übergänge sein, die zufälligerweise einen Übergang treffen, mit dem die Punktspreizfunktion hinreichend ermittelt werden kann. Ggf. müssen dabei hochfrequente Anteile, welche durch Rauschen verursacht werden, identifiziert und von der Analyse ausgeschlossen werden.

In einem weiteren Beispiel kann zusätzlich zum Messobjekt eine geeignete, im Idealfall bekannte Geometrie mit erfasst werden, z. B. eine kleine Kugel oder einen Draht. In den Projektionsdaten ist demensprechend ein geeigneter Kantenübergang vorhanden, der ausgewertet werden kann.

Gemäß einem anderen Beispiel kann das Verfahren weiter den folgenden Schritt aufweisen: Ermitteln eines Schätzwertes einer Unsicherheit einer aus den Messdaten ermittelten Messgröße des Objekts mittels der Betriebsdaten und des mindestens eines Qualitätsparameters.

Dazu können Informationen aus verschiedensten Quellen zusammengetragen werden, um die Messunsicherheit einer Messung abzuschätzen, z. B. Erfahrungswerte, eine Analyse der derzeitigen Datenqualität oder statistische Analysen der Stabilität eines Messergebnisses. Eine Überwachung des Funktionszustandes des verwendeten Computertomografiesystems kann weitere hilfreiche Informationen bereitstellen, die in die Unsicherheitsabschätzung mit einfließen können.

So kann beispielsweise aus der Überwachung bekannt sein, dass die geometrische Kalibrierung bereits eine gewisse Unsicherheit aufweist, die sich wiederum negativ auf die Messunsicherheit der durchzuführenden Messungen auswirkt. Es kann bei der Messunsicherheitsbestimmung dementsprechend ein erhöhtes "Grundniveau" der Messunsicherheit berücksichtigt werden, um realistischere Werte der Unsicherheit zu ermitteln. Hierbei kann auch berücksichtigt werden, dass sich eine Unsicherheit bzw. leichte Störung der geometrischen Kalibrierung auf unterschiedliche Messgrößen unterschiedlich auswirkt, beispielsweise stärker auf längere Messgrößeren.

In einem anderen Beispiel kann in der Überwachung ein erhöhtes Rauschniveau der Daten festgestellt werden, z. B. aufgrund einer geringeren Leistung der Röntgenröhre. Dies erhöht die Unsicherheit der Messgrößen. Hierbei kann auch die Sensitivität der einzelnen Messgrößen auf den Fehlerzustand berücksichtigt werden: die Messung einer Formabweichung reagiert beispielsweise deutlich stärker auf ein erhöhtes Rauschniveau als die Messung eines Abstands von Kugelmittelpunkten.

Während des Schritts Ermitteln von Messdaten des Objekts mittels der Vorrichtung können beispielsweise die folgenden Schritte durchgeführt werden: Ermitteln vorläufiger Messdaten und/oder mindestens eines vorläufigen Qualitätsparameters aus den Messdaten; Anpassen des Schritts Ermitteln von Messdaten des Objekts mittels der Vorrichtung unter Berücksichtigung der vorläufigen Messdaten und/oder des mindestens einen vorläufigen Qualitätsparameters aus den Messdaten.

In diesem Beispiel können die bei einer Messung anfallenden Informationen während der Messung verwendet werden, um eine Optimierung der Messung bzw. der zu verwendenden Aufnahmeparameter zu bewirken.

Das Verfahren kann gemäß einem weiteren Beispiel weiter den folgenden Schritt aufweisen: Ermitteln, ob eine aus den Messdaten ermittelte Messgröße des Objekts, vorzugsweise unter Berücksichtigung einer Unsicherheit der Messgröße, innerhalb eines vordefinierten Toleranzbereiches liegt.

Sichere Aussage bedeutet in diesem Fall, dass sich das Messergebnis, unter Berücksichtigung der Unsicherheit, entweder sicher innerhalb oder sicher außerhalb des Toleranzbereichs befindet.

Es können auf diese Weise diejenigen Bereiche identifiziert werden, in denen noch keine sichere Aussage möglich ist. Es können dann bei der Messung vornehmlich zusätzliche Informationen für diesen Bereich gesammelt werden. Für Bereiche, in denen bereits eine sichere Aussage möglich ist, ist dies nicht nötig. Dies spart Zeit bei der Messdatenaufnahme.

Weiterhin kann der Messvorgang, ggf. vorzeitig, beendet werden, wenn für alle Größen eine sichere Aussage möglich ist und/oder für eine kritische Größe die sichere Aussage möglich ist, dass die Größe außerhalb des Toleranzbereichs ist.

Die Bestimmung der Unsicherheit kann dabei Informationen aus der Überwachung verwenden.

Die Informationen aus der Überwachung, d. h. der derzeitige Zustand der Vorrichtung zum Untersuchen von Objekten, z. B. eines CT-Systems, können in einen Digital Twin der Vorrichtung fließen, um dieses aktuell bzw. möglichst identisch zur realen Vorrichtung zu halten. Dies hat Vorteile bei der Nutzung des Digital Twins, die z. B. eine simulative Messunsicherheitsbestimmung sein kann.

In einem weiteren Aspekt betrifft die Erfindung ein Computerprogrammprodukt mit auf einem Computer ausführbaren Instruktionen, welche auf einem Computer ausgeführt, den Computer dazu veranlassen, das Verfahren nach der vorhergehenden Beschreibung durchzuführen.

Vorteile und Wirkungen sowie Weiterbildungen des Computerprogrammprodukts ergeben sich aus den Vorteilen und Wirkungen sowie Weiterbildungen des oben beschriebenen Verfahrens. Es wird daher in dieser Hinsicht auf die vorangegangene Beschreibung verwiesen. Unter einem Computerprogrammprodukt kann z. B. ein Datenträger verstanden werden, auf dem ein Computerprogrammelement gespeichert ist, das für einen Computer ausführbare Instruktionen aufweist. Alternativ oder zusätzlich kann unter einem Computerprogrammprodukt beispielsweise auch ein dauerhafter oder flüchtiger Datenspeicher, wie Flash-Speicher oder Arbeitsspeicher, verstanden werden, der das Computerprogrammelement aufweist. Weitere Arten von Datenspeichern, die das Computerprogrammelement aufweisen, seien damit jedoch nicht ausgeschlossen.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: ein Flussdiagramm eines Beispiels des computerimplementierten Verfahrens mit optionalen Schritten;
- Fig. 2: ein Flussdiagramm weiterer optionaler Schritte des Verfahrens;
- Fig. 3a, b: ein Flussdiagramm von weiteren Beispielen des Verfahrens mit weiteren optionalen Schritten; und
- Fig. 4: ein Flussdiagramm eines weiteren Beispiels mit weiteren optionalen Schritten.

Im Folgenden wird das computerimplementierte Verfahren zur Zustandsüberwachung einer Vorrichtung zur Untersuchung von Objekten in seiner Gesamtheit mit dem Bezugszeichen 100 referenziert. Die Untersuchung des Objekts durch die Vorrichtung umfasst dabei das Ermitteln von Messdaten mittels einer Messung des Objekts und während der Untersuchung des Objekts das Ermitteln von Betriebsdaten der Vorrichtung.

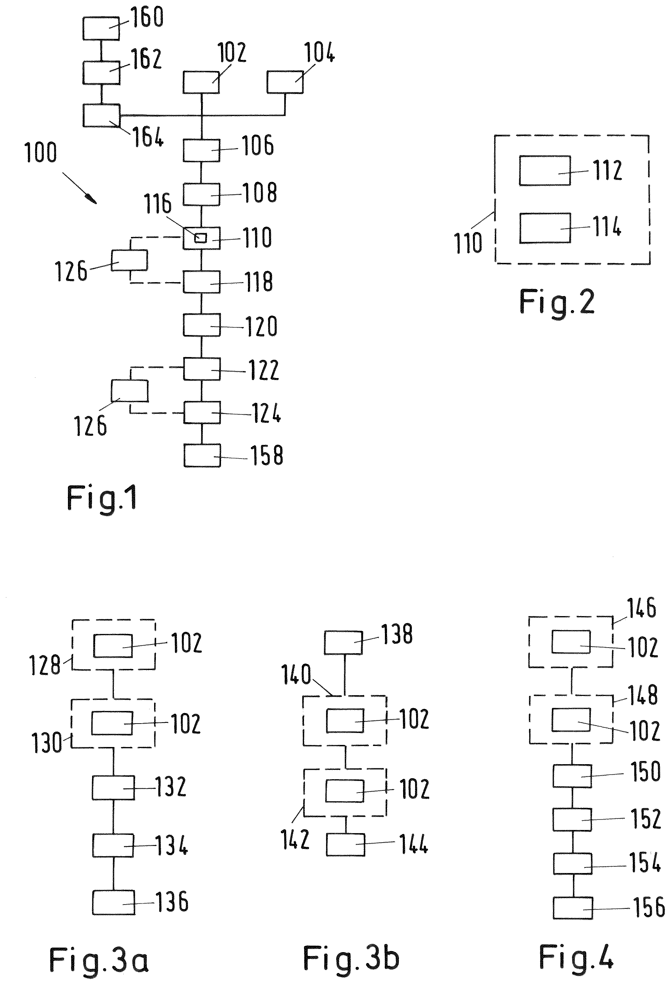

Figur 1 zeigt dabei ein Beispiel des computerimplementierten Verfahrens 100. In einem ersten Schritt 102 werden Messdaten des Objekts mittels der Vorrichtung zum Untersuchen von Objekten ermittelt. Die Messung kann hierbei zum Beispiel eine durchstrahlende Messung mittels eines Computertomographen sein. Die Messdaten können damit beispielsweise zweidimensionale Projektionsbilddaten sein. Alternativ oder zusätzlich können die Messdaten Volumenbilddaten sein, die aus dem Projektionsbilddatensatz rekonstruiert wurden, oder Oberflächendaten, die anhand der Volumenbilddaten bestimmt wurden. Wenn die Vorrichtung eine andere Messmethode bzw. einen anderen Sensor verwendet, sind entsprechend andere Daten als Messdaten definiert.

Während des Schrittes 102 wird gleichzeitig Schritt 104 durchgeführt, bei dem Betriebsdaten der Vorrichtung ermittelt werden.

Betriebsdaten der Vorrichtung können zum Beispiel Dunkelstrom und Hellstrom eines Detektors der Vorrichtung sein, d. h. eine pixelaufgelöste Information, die für den Hell-Dunkel-Abgleich bzw. Flatfield-Correction verwendet wird. Weiter können dies z. B. die Anzahl und Verteilung von defekten Pixeln des Detektors oder der Messkamera sein. Diese werden meist durch gewisse Verfahren identifiziert und als Maske gespeichert.

In Bezug auf einen Drehtisch können dies Betriebsgrößen wie Spannung und Strom sein. Weiter können dies geometrische Parameter wie Verkippung und Rundlauf sein. Es können weiter externe Sensoren, z. B. Laserinterferometer, verwendet werden. Auch "interne Sensoren" können verwendet werden, indem ein geeignetes bzw. dediziertes Objekt in verschiedenen Winkelstellungen durchstrahlt und die Abbildung auf dem Detektor ausgewertet wird. Eine spiralförmige Anordnung von Kugeln ist hierfür beispielsweise gut geeignet, da sich hier die Kugeln auf dem Detektor gegenseitig nicht abschatten und ihre Position im Strahlengang dementsprechend gut berechnet werden können. Eine solche Messung wird oft beim regelmäßig durchgeführten Einmessvorgang durchgeführt. Es können also entsprechende Eigenschaften bzw. Grö-ßen/Parameter des zuletzt durchgeführten Einmessvorgangs überwacht werden.

Hinsichtlich einer Röntgenröhre/-quelle der Vorrichtung können die Betriebsdaten die Stromkurve, die Spannungskurve, der Strom von Fokussierung und Zentrierung, oder die Differenz von Röhren- und Targetstrom sein.

Betriebsdaten können weiter z. B. Geräteinformationen sein: die Temperatur, wobei hier verschiedene Messpunkte denkbar sind, z. B. außerhalb der Vorrichtung, innerhalb einer Strahlenschutzkabine, an einem Detektor, einer Röhre, einem Drehtisch, an den Achsen, oder am Messobjekt. Auch die Tageszeit, die Dauer seit dem letzten längeren Zeitraum, in dem das Messgerät oder dessen Komponenten deaktiviert waren, die Dauer seit letztem Hell-Dunkel-Abgleich, die Zeit seit der letzten Wartung oder seit dem letzten Einmessvorgang können Betriebsdaten sein.

Weiter können grundlegende Informationen über Konfiguration, Hersteller und Bauweise des verwendeten Computertomografiesystems, z. B. Kegelstrahl, Fächerstrahl, oder Roboter-CT, als Betriebsdaten verwendet werden. Dabei kann weiter eine Berücksichtigung der bei der Messung gewählten Aufnahmeparameter, z. B. der Dauer des Scans, der Belichtungszeit pro Projektion, der Bildmittelung pro Projektion, der Nominalspannung, des Nominalstroms, der Vorfilterung, der geometrischen Vergrößerung oder der nominellen Voxelgröße als Betriebsdaten verwendet werden.

Weiter können Metainformationen über das zu messende Objekt, z. B. die Soll-Geometrie oder das Material Betriebsdaten sein.

Bei einer iterativen Rekonstruktion kann die Konvergenzgeschwindigkeit der Rekonstruktion als Betriebsdaten verwendet werden.

Aus den Messdaten wird im Schritt 106 mindestens ein Qualitätsparameter ermittelt. Qualitätsparameter kann z. B. eine ggf. lokale, Übereinstimmung zwischen den Grauwerten der Projektionen und einer Vorwärtsprojektion von rekonstruierten Volumendaten sein. Dies kann ein Maß für die Konsistenz der Volumendaten zu den Projektionsdaten sein. Wenn die Übereinstimmung gering ist, ist zu erwarten, dass Artefakte bzw. Bildfehler in den Volumendaten vorhanden sind.

Weiter kann eine Analyse des gleichmäßig ausgeleuchteten Hintergrunds in Projektionen bezüglich der Homogenität, des mittleren Grauwerts, des Signal-Rausch-Verhältnisses, oder des Noise Power Spectrums sein. Weiter kann dies die Unschärfe bzw. die Punktspreizfunktion in den Projektionen sein.

Qualitätsparameter können aus beispielsweise als Volumendaten vorliegenden Messdaten abgeleitet werden, als Analyse von Grauwerten der Volumendaten hinsichtlich ihrer Qualität in der Umgebung der Oberfläche, insbesondere kann der Fokus auf der Unschärfe bzw. die Punktspreizfunktion und die Abweichung von einem idealen Modell-Grauwertübergang liegen, da die auf diese Weise ermittelten Qualitätsparameter genau auf Basis der Daten ermittelt werden, die das Ergebnis der Oberflächenbestimmung beeinflussen, wobei die Oberfläche wiederum für viele Auswertungen wichtig ist, ist die Aussagekraft dieser Qualitätsgrößen besonders groß.

Es kann weiter eine Analyse von homogenen Bereichen der Volumendaten, z. B. den Hintergrund oder das homogene Material hinsichtlich des Signal-Rausch-Verhältnisses und der Homogenität durchgeführt werden. Diese Analyse wird dadurch erleichtert, dass durch ein Modell, im Idealfall konstanter Grauwerte im Material und im Hintergrund, Vorwissen über diese Bildbereiche existiert. Abweichungen hierzu liegen dann vermutlich in der Datenqualität und nicht an Änderungen des Objekts.

Weiter kann eine Analyse eines Grauwerthistogramms von als Volumendaten vorliegenden Messdaten durchgeführt werden, z. B. hinsichtlich des Abstands und der Breite der einzelnen Materialpeaks.

Wenn Defekte erkannt wurden, können die Volumendaten in diesen Bereichen und mit dem Wissen über die vorhandenen Defekte analysiert werden, z. B. bzgl. der Unschärfe bzw. Punktspreizfunktion oder dem Kontrast.

Weiter kann eine Analyse der aus den Volumendaten bestimmten Oberfläche durchgeführt werden, z. B. hinsichtlich Oberflächeneigenschaften wie Formabweichungen und Parametern zur Welligkeit oder Rauheit.

Die Erfassung der oben genannten Größen, sowohl der Betriebsdaten als auch der Qualitätsparameter, ist prinzipiell mit unterschiedlicher Zeitauflösung möglich, wobei die Möglichkeiten zwischen den zu überwachenden Größen variieren können. Es kann kontinuierlich, quasi-kontinuierlich mit großer Abtastrate, oder mit einer festen zeitlichen Frequenz abgetastet werden. Weiter kann eine Messgröße pro aufgenommener Projektion oder je einer vordefinierten Anzahl an aufgenommenen Projektionen erfasst werden. Weiter kann die Erfassung einer Messgröße bei speziellen Ereignissen, z. B. Start und Ende einer Messung, dem Öffnen der Schutzkabine des Computertomografiesystems, oder nach Durchführung eines Hell-Dunkel-Abgleichs durchgeführt werden.

In einem weiteren Schritt 108 werden die Betriebsdaten und der mindestens eine Qualitätsparameter analysiert. Aus der Analyse folgt, ob ein Fehlerzustand der Vorrichtung zur Untersuchung eines Objekts vorliegt.

Dazu wird im Schritt 110 ein Zustandskennwert ermittelt, der auf der Analyse der Betriebsdaten und des mindestens einen Qualitätsparameters basiert. Der Zustandskennwert gibt dabei den Zustand der Vorrichtung an. Dies kann ein Fehlerzustand oder ein Normalzustand sein. Weiter kann der Zustandskennwert auch einen oder mehrere Zustände zwischen dem Fehlerzustand und dem Normalzustand angeben. Diese Zustände zwischen dem Fehlerzustand und dem Normalzustand beschreiben uneindeutige Zustände, bei denen nicht klar ist ob es sich um einen Fehlerzustand oder einen Normalzustand handelt. Je näher ein Zwischenzustand dabei an dem Normalzustand oder dem Fehlerzustand ist, desto höher ist die Wahrscheinlichkeit, dass ein Normalzustand bzw. ein Fehlerzustand vorliegt.

Der Schritt 110 kann dabei optional einen Unterschritt 116 aufweisen, bei dem ermittelt wird, ob der Zustandskennwert einen Fehlerzustand der Vorrichtung anzeigt. D.h., bei der Ermittlung des Zustandskennwerts wird der Zustandskennwert zunächst nicht weiter ausgewertet. Erst durch das Ermitteln im Unterschritt 116, welcher Zustandskennwert nun vorliegt, wird eine Auswertung des Zustandskennwert durchgeführt.

Wenn ermittelt wurde, dass mit dem Zustandskennwert ein Zwischenzustand ermittelt wurde, kann optional mit dem Schritt 118 eine Ermittlung von Messdaten eines Referenzobjekts mittels der Vorrichtung durchgeführt werden. Dazu kann in einem weiteren optionalen Schritt 126 vorher ein Referenzobjekt für das Objekt in die Vorrichtung zur Untersuchung von Objekten eingewechselt werden, um Messdaten über das Referenzobjekt zu ermitteln. Dabei weist die Vorrichtung zur Durchführung dieses Schritts 126 eine automatische Objektwechseleinheit auf.

In einem weiteren optionalen Schritt 120 können die ermittelten Messdaten des Referenzobjekts und bei Bedarf die Betriebsdaten während der Messung des Referenzobjekts analysiert werden. Damit werden Informationen ermittelt, ob ein Fehlerzustand der Vorrichtung vorliegt. Damit können die Informationen, die der Zustandskennwert angibt, der lediglich einen Zwischenzustand anzeigen, mit dem angezeigt wird, dass ein Fehlerzustand vorliegen könnte, verbessert werden.

Aus dem Zustandskennwert kann in einem weiteren optionalen Schritt 122 ein Fehlerursachenparameter ermittelt werden. Der Fehlerursachenparameter gibt dabei eine mögliche Fehlerursache des Fehlerzustands an. Der Fehlerursachenparameter gibt jedoch in vielen Fällen keine genaue Auskunft über die Ursache des Fehlerzustands.

Wenn im Schritt 122 ein Fehlerursachenparameter ermittelt wird, der anzeigt, dass eine geometrische Kalibrierung der Vorrichtung nicht in Ordnung ist, d.h. wenn die geometrische Kalibrierung der Vorrichtung einen Genauigkeitswert aufweist, der außerhalb eines vordefinierten Genauigkeitswertintervalls liegt, wird der weitere Schritt 124 durchgeführt, bei dem die Vorrichtung kalibriert wird. Dazu kann optional der Schritt 126 durchgeführt werden, wobei die Vorrichtung dann eine automatische Objektwechseleinheit aufweist. Im Schritt 126, der für den Schritt 124 durchgeführt wird, wird ein Kalibrierobjekt für das Objekt in die Vorrichtung zur Untersuchung von Objekten eingewechselt.

Das Kalibrierobjekt weist dabei eine bekannte Geometrie auf, sodass die Vorrichtung zur Untersuchung von Objekten entsprechend kalibriert werden kann.

In einem weiteren optionalen Schritt 158 kann ein Schätzwert ermittelt werden, der eine Unsicherheit einer aus den Messdaten ermittelten Messgröße des Objekts angibt. Die Ermittlung des Schätzwertes wird auf Basis der Betriebsdaten und des mindestens einen Qualitätsparameters durchgeführt.

In einem weiteren optionalen Schritt 160 werden während des Schritts 102 vorläufige Messdaten ermittelt. Weiter wird alternativ oder zusätzlich mindestens ein vorläufiger Qualitätskennwert aus den Messdaten, die im Schritt 102 gewonnen wurden, ermittelt.

Mit den vorläufigen Messdaten bzw. mit dem vorläufigen Qualitätskennwert wird in einem weiteren optionalen Schritt 162 der Schritt 102 angepasst. Damit wird eine adaptive Messung durchgeführt, deren Messparameter während des Messvorgangs aufgrund der bisher gewonnenen Ergebnisse, die auf vorläufigen Messdaten oder auf vorläufigen Qualitätskennwerten beruhen können, verändert werden können.

Weiter kann nach den Schritten 160 und 162 der optionale Schritt 164 durchgeführt werden, bei dem ermittelt wird, ob eine aus den Messdaten ermittelten Messgröße des Objekts innerhalb eines vordefinierten Toleranzbereiches liegt. Eine Messgröße des Objekts kann zum Beispiel eine Länge einer Seite des Objekts sein. Weiter kann dies beispielsweise auch eine Dicke oder Dichte an einer bestimmten Position oder einem bestimmten Bereich im Objekt sein. Wenn die Messgröße innerhalb eines vordefinierten Toleranzbereiches liegt, wobei der vordefinierte Toleranzbereich durch eine Soll-Geometrie vorgegeben werden kann, wird die Adaption der Messung bzw. des Schritts 102 als erfolgreich angesehen.

Weiter kann der Schritt 164 unter Berücksichtigung einer Unsicherheit der Messgröße des Objekts durchgeführt werden. D.h., lediglich wenn auch unter Berücksichtigung der Unsicherheit der Messgröße diese Messgröße innerhalb des vordefinierten Toleranzbereichs liegt, wird ein Erfolg der Adaption angenommen.

Figur 2 zeigt ein Flussdiagramm des Schritts 110 mit optionalen Unterschritten 112 und 114. Die beiden Unterschritte 112 und 114 können jeweils unabhängig voneinander vorgesehen werden.

Der Unterschritt 112 betrifft einen Vergleich der Betriebsparameter mit vordefinierten Vergleichswerten für die Betriebsdaten und einen Vergleich des mindestens einen Qualitätsparameters mit vordefinierten Vergleichswerten für den mindestens ein Qualitätsparameter. Der Vergleich dient zum Ermitteln des Zustandskennwerts.

Das computerimplementierte Verfahren 100 kann mittels des Vergleichs feststellen, ob Abweichungen zwischen den Betriebsparametern und deren Vergleichswerten bzw. Abweichungen zwischen dem mindestens einen Qualitätsparameter und dessen Vergleichswert bestehen. Wenn Abweichungen bestehen, kann das ein Zeichen sein, dass eine Abweichung vom Normalzustand der Vorrichtung vorliegt. Der Zustandskennwert wird daher entweder zum Anzeigen eines Zwischenzustands oder eines Fehlerzustands gesetzt.

Der Unterschritt 114 betrifft ein Vergleich der Betriebsdaten mit vorermittelten aus Trainingsdaten abgeleiteten Musterdaten für die Betriebsdaten und einen Vergleich des mindestens einen Qualitätsparameters mit vorermittelten aus Trainingsdaten abgeleiteten Musterdaten für den mindestens einen Qualitätsparameter. Auch dieser Vergleich dient zur Ermittlung des Zustandskennwerts.

Hierbei kann das computerimplementierte Verfahren 100 mittels des Vergleichs aus Unterschritt 114 feststellen, ob Abweichungen zwischen den Betriebsparametern und den Musterdaten für die Betriebsparameter bzw. Abweichungen zwischen dem mindestens einen Qualitätsparameter und dessen Musterdaten bestehen. Die Musterdaten basieren dabei auf Trainingsdaten, mit denen vor dem Unterschritt 114 die Musterdaten ermittelt wurden. Abweichungen zwischen den Betriebsdaten und deren Musterdaten bzw. dem mindestens einen Qualitätsparameter und dessen Musterdaten indizieren eine Abweichung vom Normalzustand der Vorrichtung. Der Zustandskennwert wird daher entweder zum Anzeigen eines Zwischenzustands oder eines Fehlerzustands gesetzt.

Die Musterdaten können dabei zum Beispiel aus einer Simulation ermittelt werden, die mit den Trainingsdaten als Eingabedaten arbeitet. Dazu können Simulationstools verwendet werden, die den gesamten Mess- bzw. Durchstrahlungsprozess inklusive der Rekonstruktion und weiteren Datenauswertung realistisch nachbilden können. Auf diese Weise kann mit geringem Aufwand und zielgerichtet eine große Menge von Trainingsdaten generiert werden, in denen definierte Fehlerursachen und ihre Auswirkungen auf die Messdaten enthalten sind. Dabei können beliebige bzw. unterschiedliche Fehlerursachen, sowohl separat als auch in verschiedenen Kombinationen, simuliert werden.

Vorteil ist, dass auch die spezifischen Eigenschaften der verwendeten Vorrichtung zum Untersuchen des Objekts, z. B. eines CT-Systems, nachgebildet werden können. Dies bewirkt, dass die generierten Trainingsdaten besser zu dem überwachten CT-System passen. Es ist auch ein messaufgabenspezifisches Training möglich, indem bei den simulierten Messungen die gleichen Objekte durchstrahlt werden, die auch im realen Regelbetrieb gemessen werden sollen.

Alternativ wird zunächst wird ein Referenzobjekt untersucht und hieraus ermittelt, ob ein Fehlerzustand vorliegt. Die Geometrie des Referenzobjekts kann von der Geometrie des eigentlichen zu messenden Objekts abweichen. Hieraus können direkt Trainingsdaten ermittelt werden.

Weiterhin können auch weitere Messungen von anderen zu messenden Objekten in der zeitlichen Umgebung, d. h. kurz vor und/oder kurz nach, der Messung des zu messenden Referenzobjekts verwendet werden. Die Geometrie bzw. die Geometrien der anderen zu messenden Objekte können wiederum von der Geometrie des Referenzobjekts und des zu messenden Objekts abweichen. Außerdem muss die Geometrie der anderen Objekte nicht bekannt sein bzw. es müssen keine Referenzmessergebnisse vorliegen, um abzuschätzen zu können, ob für diese Messungen jeweils ein Fehlerzustand vorliegt. Diese Information wird aus der "zeitlich nahen" Messung des zu messenden Objekts übernommen, d. h. es wird angenommen, dass sich der Zustand der Vorrichtung in dieser kurzen Zeit nicht signifikant verändert hat.

Auf diese Weise können, ohne zusätzliche Referenzmessungen, im normalen Messbetrieb zusätzliche Trainingsdaten erzeugt werden.

Trainingsdaten können in einem anderen Beispiel generiert werden, indem die Information von Wartungen verwendet werden. Falls in durch die Wartungen festgestellt wird, dass ein gewisser Fehler vorlag, können, auch nachträglich, die Charakteristika der überwachten Daten vor diesem Zeitpunkt diesem Fehler zugeordnet werden. Weiter kann eine Analyse der Unterschiede der Charakteristika der Daten vor und nach der Wartung, in denen der Fehler behoben wurde, durchgeführt werden, um die entsprechend relevanten Charakteristika zu identifizieren. Auf diese Weise wird es ermöglicht, den entsprechenden Fehler in der Zukunft früher zu erkennen. Dazu kann z. B. vorgesehen sein, dass beispielsweise ein Hersteller von CT-Systemen über viele Wartungen auf diese Weise entsprechende Datenbanken von Trainingsdaten zur Überwachung für seine CT-Systeme, ggf. aufgeteilt nach Bauweisen, aufbaut und den Nutzern zur Verfügung stellt.

Weiterhin könne auch Fehlerzustände eines CT-System bewusst herbeigeführt werden, um ihre Auswirkungen auf die Datenqualität zu analysieren und in die Trainingsdaten aufzunehmen.

Die Figuren 3a und 3b betreffen weitere Ausführungsbeispiele des computerimplementierten Verfahrens 100, bei dem ein erstes und ein zweites Objekt gemessen werden, um Qualitätsparameter zu bestimmen. Die Geometrie des zweiten Objekts weicht dabei lediglich gering von der Geometrie des ersten Objekts ab, d. h. die Geometrie des zweiten Objekts liegt innerhalb eines vordefinierten Toleranzintervalls der Geometrie des ersten Objekts.

Gemäß Figur 3a kann das Verfahren 100 den Schritt 128 umfassen, der zumindest den Schritt 102 umfasst und mit dem ersten Objekt durchgeführt wird. Der Schritt 102 wird dabei mittels einer durchstrahlenden Messung des ersten Objekts durchgeführt. Es können jedoch weitere Schritte des Verfahrens 100 in dem Schritt 128 vorgesehen werden.

Weiter umfasst das Verfahren in diesem Beispiel den Schritt 130, der zumindest den Schritt 102 umfasst und mit dem zweiten Objekt durchgeführt wird. Der Schritt 102 wird dabei mittels einer durchstrahlenden Messung des zweiten Objekts durchgeführt. Es können jedoch weitere Schritte des Verfahrens 100 in dem Schritt 130 vorgesehen werden.

In einem weiteren Schritt 132 werden Aufnahmeparametersätze der Vorrichtung ermittelt. Für die Ermittlung der Aufnahmeparametersätze werden die als Aufnahmeparametersätze zusammengefassten Aufnahmeparameter der Messdaten des ersten Objekts und die als Aufnahmeparametersätze zusammengefassten Aufnahmeparameter der Messdaten des zweiten Objekts miteinander verglichen. Aufnahmeparametersätze, die für die Messdaten beider Objekte identisch sind, werden als die zu ermittelten Aufnahmeparametersätze ausgewählt. D.h., wenn Messdaten des ersten Objekts und Messdaten des zweiten Objekts mit identischen Aufnahmeparameter ermittelt wurden, werden die diesen Messdaten zu Grunde liegenden Aufnahmeparametersätze von dem Schritt 132 ermittelt. Wenn zum Beispiel für beide Objekte die gleiche Stellung von einem Detektor und einer Strahlungsquelle, d. h. die gleiche Durchstrahlungsgeometrie, mit den gleichen Spannungen, Strömen usw. für die Aufnahme von Messdaten verwendet wurde, sind identische Aufnahmeparameter gegeben.

In einem weiteren Schritt 134 werden die zu den ermittelten Aufnahmeparametersätzen zugehörigen Messdaten des ersten und zweiten Objekts verwendet, um erste Projektionsdarstellungen des ersten Objekts und zweite Projektionsdarstellungen des zweiten Objekts zu erzeugen. Für die ersten Projektionsdarstellungen und die zweiten Projektionsdarstellungen werden in einem weiteren Schritt 136 Qualitätsparameter ermittelt und auf Unterschiede analysiert. Für die ersten Projektionsdarstellungen werden dabei erste Qualitätsparameter und für die zweite Projektionsdarstellung zweite Qualitätsparameter ermittelt.

In einem alternativen oder zusätzlichen Beispiel des Verfahrens 100 gemäß Figur 3b können in einem Schritt 138 für die Ermittlung der Messdaten Aufnahmeparametersätze der Vorrichtung definiert werden.

Diese Aufnahmeparametersätze werden im Schritt 140 dazu verwendet, Messdaten des ersten Objekts aufzunehmen, um erste Projektionsdarstellungen zu ermitteln. Der Schritt 140 umfasst dabei zumindest den Schritt 102, der mit dem ersten Objekt durchgeführt werden. Die zu Grunde liegende Messung ist eine durchstrahlende Messung. Es können jedoch weitere Schritte des oben genannten Verfahrens 100 verwendet werden.

Im Schritt 142 werden diese Aufnahmeparametersätze dazu verwendet, Messdaten des zweiten Objekts aufzunehmen, um zweite Projektionsdarstellung zu ermitteln. Der Schritt 142 umfasst dabei zumindest den Schritt 102, der mit dem zweiten Objekt durchgeführt werden. Die zu Grunde liegende Messung ist eine durchstrahlende Messung. Es können jedoch weitere Schritte des oben genannten Verfahrens 100 verwendet werden.

Es werden weiter mindestens ein erster Qualitätsparameter der ersten Projektionsdarstellungen und mindestens ein zweiter Qualitätsparameter der zweiten Projektionsdarstellung ermittelt. Im Schritt 144 werden die ersten Qualitätsparameter und zweiten Qualitätsparameter auf Unterschiede analysiert.

Mit dem Vergleich zwischen dem ersten Qualitätsparameter und dem zweiten Qualitätsparameter kann der Funktionszustand der Vorrichtung zum Untersuchung von Objekten analysiert werden. Da jeweils Qualitätsparameter miteinander verglichen werden, denen dieselben Aufnahmeparametersätze zu Grunde liegen, sollten die Qualitätsparameter der Messdaten der beiden Objekte zumindest ähnlich sein. Bei Unterschieden weist dies darauf hin, dass sich der Funktionszustand der Vorrichtung zur Untersuchung von Objekten zwischen der Messung des ersten Objekts und des zweiten Objekts geändert hat. Die Messungen des ersten Objekts und des zweiten Objekts können einen großen zeitlichen Abstand zueinander aufweisen. D.h., es kann eine Änderung des Funktionszustands über einen großen Zeitraum untersucht werden.

Figur 4 zeigt ein weiteres alternatives oder zusätzliches Ausführungsbeispiel des computerimplementierten Verfahrens 100, bei dem ein erstes und ein zweites Objekt gemessen werden, um den Funktionszustand der Vorrichtung zur Untersuchung von Objekten zu analysieren.

In einem Schritt 146 umfasst dieses Beispiel zumindest den Schritt 102, der mit dem ersten Objekt durchgeführt wird. Weitere Schritte des Verfahrens 100 können jedoch ebenfalls vorgesehen werden. Die verwendete Messung ist eine durchstrahlende Messung.

In einem Schritt 148 umfasst dieses Beispiel zumindest den Schritt 102, der mit dem zweiten Objekt durchgeführt wird. Weitere Schritte des Verfahrens 100 können jedoch ebenfalls vorgesehen werden. Die verwendete Messung ist eine durchstrahlende Messung. Für das zweite Objekt wird im Schritt 102 eine andere Durchstrahlungsgeometrie verwendet als für das erste Objekt.

Im Schritt 150 werden aus den Messdaten des ersten Objekts Projektionsdarstellungen ermittelt. Weiter werden im Schritt 152 aus den Messdaten des zweiten Objekts ebenfalls Projektionsdarstellungen ermittelt.

Die Projektionsdarstellungen des ersten Objekts und die Projektionsdarstellung des zweiten Objekts werden analysiert und jeweils eine Unschärfe der Projektionsdarstellungen ermittelt. Im Schritt 154 wird die Unschärfe der Projektionsdarstellungen des ersten Objekts und die Unschärfe der Produktionsdarstellung des zweiten Objekts miteinander verglichen.

In einem Schritt 156 werden die Unschärfe der ersten Projektionsdarstellung und die Unschärfe der zweiten Projektionsdarstellung analysiert, um den Zustandskennwert zu ermitteln. Da die Unschärfe von Projektionsdarstellungen über viele Messungen vergleichbar bleibt, unabhängig davon wie die Geometrie des durchstrahlten Objekts ist, kann bei einer Veränderung der Unschärfe zwischen den beiden Projektionsdarstellungen auf eine Veränderung der restlichen zwischen den Messungen variierenden Einflussgrößen geschlossen werden. Auf diese Weise kann ein Fehlerzustand erkannt werden.

Das computerimplementierte Verfahren 100 kann mittels eines Computerprogrammprodukts auf einem Computer ausgeführt werden. Das Computerprogrammprodukt weist dabei auf einem Computer ausführbaren Instruktionen auf. Wenn diese Instruktionen auf einem Computer ausgeführt werden, veranlassen sie den Computer dazu, das Verfahren durchzuführen.

Die Erfindung ist nicht auf eine der vorbeschriebenen Ausführungsformen beschränkt, sondern durch den beiliegenden Anspruchssatz definiert.

## Patentansprüche

1. Computerimplementiertes Verfahren zur Zustandsüberwachung einer Vorrichtung zur Untersuchung von Objekten, wobei die Untersuchung eines Objekts das Ermitteln von Messdaten mittels einer Messung des Objekts umfasst und während der Untersuchung des Objekts Betriebsdaten der Vorrichtung ermittelt werden, wobei das Verfahren (100) die folgenden Schritte aufweist:
- Ermitteln (102) von Messdaten des Objekts mittels der Vorrichtung;
- Ermitteln (104) von Betriebsdaten der Vorrichtung während des Schritts Ermitteln (102) von Messdaten des Objekts;
- Ermitteln (106) mindestens eines Qualitätsparameters aus den Messdaten;
- Analysieren (108) der Betriebsdaten und des mindestens einen Qualitätsparameters; und
- Ermitteln (110) eines Zustandskennwerts basierend auf der Analyse der Betriebsdaten und des mindestens einen Qualitätsparameters zur Zustandsüberwachung der Vorrichtung, wobei der Zustandskennwert einen Zustand der Vorrichtung angibt,
wobei das Verfahren (100) weiter die folgenden Schritte aufweist:
- Durchführen (146) zumindest des Schritts Ermitteln (102) von Messdaten des Objekts mit einem ersten Objekt, wobei die Messung eine durchstrahlende Messung ist;
- Durchführen (148) zumindest des Schritts Ermitteln (102) von Messdaten des Objekts mit einem zweiten Objekt, wobei die Messung eine durchstrahlende Messung ist, wobei eine andere Durchstrahlungsgeometrie verwendet wird als für das erste Objekt;
- Ermitteln (150) von Projektionsdarstellungen aus den Messdaten des ersten Objekts;
- Ermitteln (152) von Projektionsdarstellungen aus den Messdaten des zweiten Objekts;
- Vergleichen (154) mindestens einer Unschärfe zwischen den Projektionsdarstellungen des ersten Objekts und den Projektionsdarstellungen des zweiten Objekts; und
- Analysieren (156) der mindestens einen Unschärfe zur Ermittlung des Zustandskennwerts.

2. Verfahren nach Anspruch 1, **dadurch gekennzeichnet, dass** der Schritt Ermitteln (110) eines Zustandskennwerts den folgenden Unterschritt aufweist:
- Vergleichen (112) der Betriebsdaten und des mindestens einen Qualitätsparameters mit vordefinierten Vergleichswerten für die Betriebsdaten und den mindestens einen Qualitätsparameter zur Ermittlung eines Zustandskennwerts.

3. Verfahren nach einem der Ansprüche 1 oder 2, **dadurch gekennzeichnet, dass** der Schritt Ermitteln (110) eines Zustandskennwerts weiter den folgenden Unterschritt aufweist:
- Vergleichen (114) der Betriebsdaten und des mindestens einen Qualitätsparameters mit vorermittelten aus Trainingsdaten abgeleiteten Musterdaten für die Betriebsdaten und den mindestens einen Qualitätsparameter zur Ermittlung eines Zustandskennwerts.

4. Verfahren nach Anspruch 3, **dadurch gekennzeichnet, dass** die Trainingsdaten aus Messdaten erzeugt werden, die mittels der Vorrichtung mittels mindestens einer Messung innerhalb eines vordefinierten Zeitintervalls vor und/oder nach dem Ermitteln (102) von Messdaten eines Referenzobjekts ermittelt werden, wobei das Referenzobjekt eine bekannte Geometrie und/oder eine bekannte Eigenschaft aufweist.

5. Verfahren nach einem der Ansprüche 1 bis 4, **dadurch gekennzeichnet, dass** der Schritt Ermitteln (110) eines Zustandskennwerts weiter den folgenden Unterschritt aufweist:
- Ermitteln (116), ob der Zustandskennwert einen Fehlerzustand der Vorrichtung anzeigt.

6. Verfahren nach Anspruch 5, **dadurch gekennzeichnet, dass** das Verfahren (100) weiter den folgenden Schritt aufweist, wenn der Zustandskennwert anzeigt, dass ein Fehlerzustand der Vorrichtung vorliegen könnte:
- Ermitteln (118) von Messdaten eines Referenzobjekts mittels der Vorrichtung; und
- Analysieren (120) der Messdaten des Referenzobjekts zum Ermitteln von Informationen, ob der Fehlerzustand der Vorrichtung vorliegt.

7. Verfahren nach einem der Ansprüche 1 bis 6, **dadurch gekennzeichnet, dass** das Verfahren (100) weiter den folgenden Schritt aufweist:
- Ermitteln (122) eines Fehlerursachenparameters zumindest aus dem Zustandskennwert, wobei der Fehlerursachenparameter eine mögliche Fehlerursache für einen Fehlerzustand angibt.

8. Verfahren nach Anspruch 7 , **dadurch gekennzeichnet, dass**, wenn im Schritt Ermitteln (122) eines Fehlerursachenparameters ein Fehlerursachenparameter ermittelt wird, der anzeigt, dass eine geometrische Kalibrierung der Vorrichtung einen Genauigkeitswert aufweist, der außerhalb eines vordefinierten Genauigkeitswertintervalls angeordnet ist, das Verfahren (100) weiter folgenden Schritt aufweist:
- Kalibrieren (124) der Vorrichtung.

9. Verfahren nach Anspruch 6 oder 8, **dadurch gekennzeichnet, dass** die Vorrichtung eine automatische Objektwechseleinheit aufweist, wobei das Verfahren (100) weiter den folgenden Schritt aufweist:
- Einwechseln (126) eines Referenzobjekts für das Objekt in die Vorrichtung zur Untersuchung von Objekten zum Ermitteln von Messdaten über das Referenzobjekt und/oder Einwechseln eines Kalibrierobjektes für das Objekt in die Vorrichtung zur Untersuchung von Objekten zum Kalibrieren der Vorrichtung zur Untersuchung von Objekten.

10. Verfahren nach einem der Ansprüche 1 bis 9, **dadurch gekennzeichnet, dass** das Verfahren (100) weiter die folgenden Schritte aufweist:
- Durchführen (128) zumindest des Schritts Ermitteln (102) von Messdaten des Objekts mit einem ersten Objekt, wobei die Messung eine durchstrahlende Messung ist;
- Durchführen (130) zumindest des Schritts Ermitteln (102) von Messdaten des Objekts mit einem zweiten Objekt, wobei die Messung eine durchstrahlende Messung ist;
- Ermitteln (132) von Aufnahmeparametersätzen der Vorrichtung, die für die Ermittlung von Messdaten für das erste Objekt und für die Ermittlung von Messdaten für das zweite Objekt identisch sind;
- Ermitteln (134) von ersten Projektionsdarstellungen aus den Messdaten für das erste Objekt mittels der ermittelten Aufnahmeparametersätze und Ermitteln von zweiten Projektionsdarstellungen aus den Messdaten für das zweite Objekt mittels der ermittelten Aufnahmeparametersätze; und
- Analysieren (136) mindestens eines ersten Qualitätsparameters, der einer den ersten Projektionsdarstellungen zugeordnet ist, und mindestens eines zweiten Qualitätsparameters, der mindestens einer der zweiten Projektionsdarstellungen zugeordnet ist, auf Unterschiede;
und/oder
dass das Verfahren (100) weiter die folgenden Schritte aufweist, wenn ein Aufnahmeparametersatz eines ersten Objekts und ein Aufnahmeparametersatz eines zweiten Objekts zumindest teilweise nicht identisch sind:
- Definieren (138) von Aufnahmeparametersätzen der Vorrichtung für die Ermittlung von Messdaten;
- Durchführen (140) zumindest des Schritts Ermitteln (102) von Messdaten des Objekts mit dem ersten Objekt mit den definierten Aufnahmeparametersätzen zum Ermitteln von ersten Projektionsdarstellungen, wobei die Messung eine durchstrahlende Messung ist;
- Durchführen (142) zumindest des Schritts Ermitteln (102) von Messdaten des Objekts mit einem zweiten Objekt, mit den definierten Aufnahmeparametersätzen zum Ermitteln von zweiten Projektionsdarstellungen, wobei die Messung eine durchstrahlende Messung ist; und
- Analysieren (144) mindestens eines ersten Qualitätsparameters, der einer der ersten Projektionsdarstellungen zugeordnet ist, und mindestens eines zweiten Qualitätsparameters, der mindestens einer der zweiten Projektionsdarstellungen zugeordnet ist, auf Unterschiede;
wobei eine Geometrie des zweiten Objekts innerhalb eines vordefinierten Toleranzintervalls von einer Geometrie des ersten Objekts abweicht.

11. Verfahren nach einem der Ansprüche 1 bis 10, **dadurch gekennzeichnet, dass** das Verfahren (100) weiter den folgenden Schritt aufweist:
- Ermitteln (158) eines Schätzwertes einer Unsicherheit einer aus den Messdaten ermittelten Messgröße des Objekts mittels der Betriebsdaten und des mindestens eines Qualitätsparameters.

12. Verfahren nach einem der Ansprüche 1 bis 11, **dadurch gekennzeichnet, dass** während des Schritts Ermitteln (102) von Messdaten des Objekts mittels der Vorrichtung die folgenden Schritte durchgeführt werden:
- Ermitteln (160) vorläufiger Messdaten und/oder mindestens eines vorläufigen Qualitätsparameters aus den Messdaten;
- Anpassen (162) des Schritts Ermitteln (102) von Messdaten des Objekts mittels der Vorrichtung unter Berücksichtigung der vorläufigen Messdaten und/oder des mindestens einen vorläufigen Qualitätsparameters aus den Messdaten.

13. Verfahren nach Anspruch 12, **dadurch gekennzeichnet, dass** das Verfahren (100) weiter den folgenden Schritt aufweist:
- Ermitteln (164), ob eine aus den Messdaten ermittelte Messgröße des Objekts, vorzugsweise unter Berücksichtigung einer Unsicherheit der Messgröße, innerhalb eines vordefinierten Toleranzbereiches liegt.

14. Computerprogrammprodukt mit auf einem Computer ausführbaren Instruktionen, welche auf einem Computer ausgeführt den Computer und eine Vorrichtung zur Untersuchung von Objekten dazu veranlassen, das Verfahren nach einem der vorhergehenden Ansprüche durchzuführen.

## Claims

1. Computer-implemented method for monitoring the state of a device for examining objects, wherein the examination of an object comprises determining measurement data by way of a measurement of the object and operating data of the device are determined during the examination of the object, wherein the method (100) comprises the following steps:
- determining (102) measurement data of the object by way of the device;
- determining (104) operating data of the device during the step of determining (102) measurement data of the object;
- determining (106) at least one quality parameter from the measurement data;
- analysing (108) the operating data and the at least one quality parameter; and
- determining (110) a state characteristic value based on the analysis of the operating data and the at least one quality parameter for monitoring the state of the device, wherein the state characteristic value indicates a state of the device,
wherein the method (100) further comprises the following steps:
- carrying out (146) at least the step of determining (102) measurement data of the object with a first object, wherein the measurement is a radiographic measurement;
- carrying out (148) at least the step of determining (102) measurement data of the object with a second object, wherein the measurement is a radiographic measurement, wherein a radiographic geometry used is different from that used for the first object;
- determining (150) projection representations from the measurement data of the first object;
- determining (152) projection representations from the measurement data of the second object;
- comparing (154) at least one blur between the projection representations of the first object and the projection representations of the second object; and
- analysing (156) the at least one blur in order to determine the state characteristic value.

2. Method according to Claim 1, **characterized in that** the step of determining (110) a state characteristic value comprises the following sub-step:
- comparing (112) the operating data and the at least one quality parameter with predefined comparison values for the operating data and the at least one quality parameter for determining a state characteristic value.

3. Method according to either of Claims 1 and 2, **characterized in that** the step of determining (110) a state characteristic value further comprises the following sub-step:
- comparing (114) the operating data and the at least one quality parameter with predetermined sample data derived from training data for the operating data and the at least one quality parameter for determining a state characteristic value.

4. Method according to Claim 3, **characterized in that** the training data are generated from measurement data which are determined by way of the device by way of at least one measurement within a predefined time interval before and/or after determining (102) measurement data of a reference object, wherein the reference object has a known geometry and/or a known property.

5. Method according to any of Claims 1 to 4, **characterized in that** the step of determining (110) a state characteristic value further comprises the following sub-step:
- determining (116) whether the state characteristic value indicates a fault state of the device.

6. Method according to Claim 5, **characterized in that** the method (100) further comprises the following step if the state characteristic value indicates that a fault state of the device might be present:
- determining (118) measurement data of a reference object by way of the device; and
- analysing (120) the measurement data of the reference object in order to determine whether the fault state of the device is present.

7. Method according to any of Claims 1 to 6, **characterized in that** the method (100) further comprises the following step:
- determining (122) a fault cause parameter at least from the state characteristic value, wherein the fault cause parameter indicates a possible fault cause for a fault state.

8. Method according to Claim 7, **characterized in that**, if, in the step of determining (122) a fault cause parameter, a fault cause parameter is determined which indicates that a geometric calibration of the device has an accuracy value which is disposed outside a predefined accuracy value interval, the method (100) further comprises the following step:
- calibrating (124) the device.

9. Method according to Claim 6 or 8, **characterized in that** the device comprises an automatic object changing unit, wherein the method (100) further comprises the following step:
- substituting (126) a reference object for the object into the device for examining objects in order to determine measurement data about the reference object and/or substituting a calibration object for the object into the device for examining objects in order to calibrate the device for examining objects.

10. Method according to any of Claims 1 to 9, **characterized in that** the method (100) further comprises the following steps:
- carrying out (128) at least the step of determining (102) measurement data of the object with a first object, wherein the measurement is a radiographic measurement;
- carrying out (130) at least the step of determining (102) measurement data of the object with a second object, wherein the measurement is a radiographic measurement;
- determining (132) recording parameter sets of the device which are identical for the determination of measurement data for the first object and for the determination of measurement data for the second object;
- determining (134) first projection representations from the measurement data for the first object by way of the determined recording parameter sets and determining second projection representations from the measurement data for the second object by way of the determined recording parameter sets; and
- analysing (136) at least one first quality parameter assigned to one of the first projection representations and at least one second quality parameter assigned to at least one of the second projection representations, for differences;
and/or
**in that** the method (100) further comprises the following steps if a recording parameter set of a first object and a recording parameter set of a second object are at least partly not identical:
- defining (138) recording parameter sets of the device for the determination of measurement data;
- carrying out (140) at least the step of determining (102) measurement data of the object with the first object with the defined recording parameter sets for determining first projection representations, wherein the measurement is a radiographic measurement;
- carrying out (142) at least the step of determining (102) measurement data of the object with a second object with the defined recording parameter sets for determining second projection representations, wherein the measurement is a radiographic measurement; and
- analysing (144) at least one first quality parameter assigned to one of the first projection representations and at least one second quality parameter assigned to at least one of the second projection representations, for differences;
wherein a geometry of the second object deviates from a geometry of the first object within a predefined tolerance interval.

11. Method according to any of Claims 1 to 10, **characterized in that** the method (100) further comprises the following step:
- determining (158) an estimated value of an uncertainty of a measurement variable of the object determined from the measured data by way of the operating data and the at least one quality parameter.

12. Method according to any of Claims 1 to 11, **characterized in that**, during the step of determining (102) measurement data of the object by way of the device, the following steps are carried out:
- determining (160) preliminary measurement data and/or at least one preliminary quality parameter from the measurement data;
- adapting (162) the step of determining (102) measurement data of the object by way of the device taking into account the preliminary measurement data and/or the at least one preliminary quality parameter from the measurement data.

13. Method according to Claim 12, **characterized in that** the method (100) further comprises the following step:
- determining (164) whether a measurement variable of the object determined from the measurement data, preferably taking into account an uncertainty of the measurement variable, lies within a predefined tolerance range.

14. Computer program product comprising instructions which are executable on a computer and which, when executed on a computer, cause the computer and a device for examining objects to carry out the method according to any of the preceding claims.

## Revendications

1. Procédé mis en œuvre par ordinateur permettant de surveiller l'état d'un dispositif d'examen d'objets, l'examen d'un objet consistant à déterminer de données de mesure au moyen d'une mesure de l'objet, et des données de fonctionnement du dispositif étant déterminées lors de l'examen de l'objet, le procédé (100) comprenant les étapes consistant à :
- déterminer (102) des données de mesure de l'objet au moyen du dispositif ;
- déterminer (104) des données de fonctionnement du dispositif au cours de l'étape de détermination (102) de données de mesure de l'objet ;
- déterminer (106) au moins un paramètre de qualité à partir des données de mesure ;
- analyser (108) les données de fonctionnement et ledit au moins un paramètre de qualité ; et
- déterminer (110) une valeur caractéristique d'état sur la base de l'analyse des données de fonctionnement et dudit au moins un paramètre de qualité afin de surveiller l'état du dispositif, la valeur caractéristique d'état indiquant un état du dispositif,
le procédé (100) comprenant en outre les étapes consistant à :
- exécuter (146) au moins l'étape de détermination (102) de données de mesure de l'objet avec un premier objet, la mesure étant une mesure par transmission ;
- exécuter (148) au moins l'étape de détermination (102) de données de mesure de l'objet avec un deuxième objet, la mesure étant une mesure par transmission, une géométrie de transmission différente de celle utilisée pour le premier objet étant utilisée ;
- déterminer (150) des représentations par projection à partir des données de mesure du premier objet ;
- déterminer (152) des représentations par projection à partir des données de mesure du deuxième objet ;
- comparer (154) au moins un flou entre les représentations par projection du premier objet et les représentations par projection du deuxième objet ; et
- analyser (156) ledit au moins un flou pour déterminer la valeur caractéristique d'état.

2. Procédé selon la revendication 1, **caractérisé en ce que** l'étape de détermination (110) d'une valeur caractéristique d'état comprend la sous-étape consistant à :
- comparer (112) les données de fonctionnement et ledit au moins un paramètre de qualité à des valeurs de comparaison prédéfinies pour les données de fonctionnement et ledit au moins un paramètre de qualité afin de déterminer une valeur caractéristique d'état.

3. Procédé selon l'une quelconque des revendications 1 ou 2, **caractérisé en ce que** l'étape de détermination (110) d'une valeur caractéristique d'état comprend en outre la sous-étape consistant à :
- comparer (114) les données de fonctionnement et ledit au moins un paramètre de qualité à des données de modèle prédéterminées dérivées de données d'apprentissage pour les données de fonctionnement et ledit au moins un paramètre de qualité afin de déterminer une valeur caractéristique d'état.

4. Procédé selon la revendication 3, **caractérisé en ce que** les données d'apprentissage sont générées à partir de données de mesure qui sont déterminées au moyen du dispositif à l'aide d'au moins une mesure effectuée au cours d'un intervalle de temps prédéfini avant et/ou après la détermination (102) de données de mesure d'un objet de référence, l'objet de référence présentant une géométrie connue et/ou une propriété connue.

5. Procédé selon l'une quelconque des revendications 1 à 4, **caractérisé en ce que** l'étape de détermination (110) d'une valeur caractéristique d'état comprend en outre la sous-étape consistant à :
- déterminer (116) si la valeur caractéristique d'état indique un état de défaillance du dispositif.

6. Procédé selon la revendication 5, **caractérisé en ce que** le procédé (100) comprend en outre l'étape consistant, lorsque la caractéristique d'état indique qu'un état de défaillance du dispositif pourrait se produire, à :
- déterminer (118) des données de mesure d'un objet de référence au moyen du dispositif ; et
- analyser (120) les données de mesure de l'objet de référence afin de déterminer si l'état de défaillance du dispositif se produit.

7. Procédé selon l'une quelconque des revendications 1 à 6, **caractérisé en ce que** le procédé (100) comprend en outre l'étape consistant à :
- déterminer (122) un paramètre de cause de défaillance au moins à partir de la caractéristique d'état, le paramètre de cause de défaillance indiquant une cause de défaillance possible pour un état de défaillance.

8. Procédé selon la revendication 7, **caractérisé en ce que**, lorsque l'étape de détermination (122) d'un paramètre de cause de défaillance détermine un paramètre de cause de défaillance indiquant qu'un étalonnage géométrique du dispositif présente une valeur de précision qui se situe en dehors d'un intervalle de valeurs de précision prédéfini, le procédé (100) comprend en outre l'étape consistant à :
- étalonner (124) le dispositif.

9. Procédé selon la revendication 6 ou 8, **caractérisé en ce que** le dispositif comporte une unité de changement automatique d'objets, le procédé (100) comprenant en outre l'étape consistant à :
- remplacer (126) l'objet par un objet de référence dans le dispositif d'examen d'objets pour déterminer des données de mesure concernant l'objet de référence et/ou remplacer l'objet par un objet d'étalonnage dans le dispositif d'examen d'objets pour étalonner le dispositif d'examen d'objets.

10. Procédé selon l'une quelconque des revendications 1 à 9, **caractérisé en ce que** le procédé (100) comprend en outre les étapes consistant à :
- exécuter (128) au moins l'étape de détermination (102) de données de mesure de l'objet avec un premier objet, la mesure étant une mesure par transmission ;
- exécuter (130) au moins l'étape de détermination (102) de données de mesure de l'objet avec un deuxième objet, la mesure étant une mesure par transmission ;
- déterminer (132) des ensembles de paramètres d'acquisition du dispositif qui sont identiques pour la détermination de données de mesure pour le premier objet et pour la détermination de données de mesure pour le deuxième objet ;
- déterminer (134) des premières représentations par projection à partir des données de mesure pour le premier objet au moyen des ensembles de paramètres d'acquisition déterminés et déterminer des deuxièmes représentations par projection à partir des données de mesure pour le deuxième objet au moyen des ensembles de paramètres d'acquisition déterminés ; et
- analyser (136) au moins un premier paramètre de qualité qui est associé à l'une des premières représentations par projection, et au moins un deuxième paramètre de qualité qui est associé à au moins l'une des deuxièmes représentations par projection pour déterminer des différences ;
et/ou
**en ce que** le procédé (100) comprend en outre les étapes consistant, lorsqu'un ensemble de paramètres d'acquisition d'un premier objet et un ensemble de paramètres d'acquisition d'un deuxième objet ne sont pas au moins en partie identiques, à :
- définir (138) des ensembles de paramètres d'acquisition du dispositif pour la détermination de données de mesure ;
- exécuter (140) au moins l'étape de détermination (102) de données de mesure de l'objet avec le premier objet à l'aide des ensembles de paramètres d'acquisition définis, pour déterminer des premières représentations par projection, la mesure étant une mesure par transmission ;
- exécuter (142) au moins l'étape de détermination (102) de données de mesure de l'objet avec un deuxième objet à l'aide des ensembles de paramètres d'acquisition définis, pour déterminer des deuxièmes représentations par projection, la mesure étant une mesure par transmission ; et
- analyser (144) au moins un premier paramètre de qualité qui est associé à l'une des premières représentations par projection, et au moins un deuxième paramètre de qualité qui est associé à au moins l'une des deuxièmes représentations par projection pour déterminer des différences ;
une géométrie du deuxième objet s'écartant d'une géométrie du premier objet dans un intervalle de tolérance prédéfini.

11. Procédé selon l'une quelconque des revendications 1 à 10, **caractérisé en ce que** le procédé (100) comprend en outre l'étape consistant à :
- déterminer (158) une estimation d'une incertitude d'une grandeur de mesure de l'objet déterminée à partir des données de mesure au moyen des données de fonctionnement et dudit au moins un paramètre de qualité.

12. Procédé selon l'une quelconque des revendications 1 à 11, **caractérisé en ce que**, lors de l'étape de détermination (102) de données de mesure de l'objet au moyen du dispositif, les étapes suivantes sont exécutées :
- déterminer (160) des données de mesure provisoires et/ou au moins un paramètre de qualité provisoire à partir des données de mesure ;
- adapter (162) l'étape de détermination (102) de données de mesure de l'objet au moyen du dispositif en tenant compte des données de mesure provisoires et/ou dudit au moins un paramètre de qualité provisoire à partir des données de mesure.

13. Procédé selon la revendication 12, **caractérisé en ce que** le procédé (100) comprend en outre l'étape consistant à :
- déterminer (164) si un paramètre de mesure de l'objet déterminé à partir des données de mesure, de préférence en tenant compte d'une incertitude du paramètre de mesure, se situe dans une plage de tolérance prédéfinie.

14. Produit programme d'ordinateur comprenant des instructions exécutables sur un ordinateur qui, lorsqu'elles sont exécutées sur un ordinateur, amènent l'ordinateur et un dispositif d'examen d'objets à mettre en œuvre le procédé selon l'une quelconque des revendications précédentes.
